# EUROPEAN PATENT APPLICATION

(11) **EP 4 670 651 A1**
(43) Date of publication of application: **31.12.2025**
(21) Application number: 24315310.3
(22) Date of filing: 27.06.2024
(51) Int. Cl.: A61B 17/22, A61B 17/00, A61M 25/01

(54) **CATHETER DEVICE FOR RETRIEVING A THROMBUS FROM A VESSEL**

(71) Applicant: Artedrone, 75013 Paris (FR)
(72) Inventor: POUPONNEAU, Pierre, 44300 Nantes (FR); GRIMAUD, Michel, 78000 Versailles (FR); POULETTY, Philippe, 75005 Paris (FR); JAGUENAUD, Morgane, 78810 Feucherolles (FR)
(74) Representative: Hepp Wenger Ryffel AG

(57) **Abstract**

The invention relates to a catheter device (1) for retrieving a thrombus (T) from a vessel. The catheter device comprises a main body (15) with an internal channel (15') extending at least through a distal part of the main body (15). An attachment element (4) is optionally attached or attachable to the main body (15) by a flexible line (2). The attachment element (4) further comprises a magnetic element (7) for steering and/or guiding by an external magnetic actuator and is adapted to attach to the thrombus (T) by means of a suction mechanism. The suction mechanism may include a suction hole (8) and /or a suction line (3). The suction mechanism may comprise a suction cup (6). The thrombus (T) is retrievable with the catheter device by exerting a pulling force on the attachment element (4) via the flexible line (2).

## Description

The present invention relates to a catheter device according to the preamble of the independent claims.

The retrieving of a thrombus in the case of an ischemic accident has to be done as soon as possible. The general procedure may be done with catheters inserted from the femoral artery.

The catheter may be pushed manually up to the cerebral network with the use of a guidewire. Retrieving tool such as an aspiration catheters and stent retrievers may be used. However, the navigation of the catheter, especially in tortuous vessels in aged patients, remains challenging and lengthy.

It is known in the prior art to use medical devices to remove thrombi from vessels.

For example, US 2017/119407 discloses aspiration devices for removal of blood clots.

US 2013/060269 discloses a stent device which penetrates a material in order to anchor it.

US 2013/289578 discloses a catheter device for surrounding and gripping blood clots.

WO 2020/064663 discloses microrobots for treatment of vessels.

WO 2021/198411 discloses a microrobot with a suction mechanism for treatment of vessels.

US 2012/0041475 discloses thrombus management devices for the treatment of acute ischemic strokes.

However, devices and methods known in the art have several drawbacks. For example, known devices are typically large, which may limit the accessible locations in a vessel system and increase the risk of unwanted interaction with tissue which may lead to injuries. Furthermore, large devices may be more difficult to operate by a surgeon. Furthermore, known devices may require complicated mechanisms for safe removal of a thrombus. In addition, treatments using known devices and methods may take a long time, leading to higher patient discomfort and higher risks associated with the treatment.

Thus, the object of the present invention is to overcome the drawbacks of the prior art, in particular to provide a device, a system and a method to provide easy, safe and versatile treatment of vessels. In particular, it is an object to improve the navigation and the retrieving time of thrombi in vessels.

These and other objects are achieved by the catheter device according to the characterizing portion of the independent claims of the invention.

The catheter device according to the invention is adapted for retrieving a thrombus from a vessel. The catheter device comprises a main body having an internal channel which extends at least through a distal portion of the main body. The catheter device further comprises an attachment element which is adapted to attach to the thrombus by means of a suction mechanism. Preferably, the suction mechanism includes a suction hole and/or a suction line. The attachment mechanism may be adapted for attachment to a proximal face of the thrombus. Particularly preferably, the suction mechanism includes a suction cup which includes the suction hole. The suction hole may be in fluid communication with the suction line. The attachment element is formed by a distal part of the catheter device and is, optionally, attached or attachable to the main body of the catheter device by a flexible line. The attachment element comprises a magnetic element for steering and/or guiding by an external magnetic actuator.

The attachment element may, in some embodiments, comprise a suction cup having a suction hole and being adapted to attach to the thrombus by means of a suction mechanism, in particular to a proximal face of a thrombus. As a result, the thrombus may be retrievable by exerting a pulling force on the attachment element via the flexible line.

The suction cup may allow to attach the device to the thrombus. The magnetic element may allow to orientate and to attract the distal part of the device.

In the most general terms, the catheter device according to the invention enables fast navigation through larger parts of the vasculature. However, navigation through tortuous structures is challenging and may become slower. Therefore, once it is in closer proximity to a treatment site, typically a thrombus, a distal part connected by a flexible line may be released from the catheter body and navigated to a treatment site by magnetic forces. Navigation, e.g. by magnetic forces and flow in blood, may be slower than pushing of the catheter generally, but may allow more precise positioning and orientation and may even be faster than pushing of a catheter in small and/or tortuous structures, thus allowing better access to such structures. Therefore, the catheter device provides particularly fast and versatile treatment options. Furthermore, a retrieved thrombus may be pulled into the catheter main body in order to retrieve it, thus making the treatment safer by minimizing the time and distance travelled by the thrombus while being pulled back in the blood stream.

The device according to the invention may move autonomously at least based on a force or a combination of forces in the vascular network up to the thrombus. The device may anchor to a proximal surface of the thrombus by suction and retrieve it by moving backwards. The device may be controlled by an external system.

The device may in particular comprise at least a suction cup, a magnetic element, a flexible hollow line and a pushing element.

It will be understood that in some embodiments, the flexible line may not be moved by a flow because of its moderate bending stiffness. The line may be bent by the magnetic element. The progressive displacement of the line is achieved by combining a proximal pushing and a magnetic attraction induced by an external magnetic field.

The magnetic force required to drag the flexible line may be reduced in narrow blood vessels, in particular when the diameter of the vessel is in the range of two times the diameter of the flexible line.

A pushing element may be connected to the flexible line. The pushing element may be formed by a hollow tube which does not collapse when it is pushed from the proximal side. Its design allows the connection to its proximal side with a vacuum pump. The pushing element may be moved by an activator such as a linear actuator or wheels. The pushing element may have an inner diameter between 0.2 mm and 2.5 mm, preferably between 0.6 mm and 1.5 mm. The outer diameter may be between 0.5 mm and 3 mm, preferably between 0.8 mm and 1.8 mm. The diameter of the section of the pushing element may increase progressively from the distal part to the proximal part. For example, the distal part may have an inner diameter of 0.7 mm and an outer diameter of 1 mm, increasing progressively towards ae proximal part having an inner diameter of 1.1 mm and an outer diameter of 1.5 mm. The proximal part of the pushing element may have a shape which optimizes the connection to a valve or a device. For example, the proximal part may provide a male Luer connection.

The suction cup may comprise or consist of titanium, nickel-titanium alloys, and/or a polymer, in particular an elastomer (e.g. silicone), polyurethane, polyether-block-amide, polyesters or a combination of polymers or elastomers. The suction cup may also comprise or consist of composite materials, e.g. a polymer matrix reinforced with particles and/or fibers to provide specific material properties such as anisotropy.

Typically, the suction cup has tensile strain values at 100% elongation between 0.05 MPa and 50 MPa and hardness values between shore A0 and D100.

The length of the suction cup in a direction corresponding to the main axis of the distal part may range between 0.1 mm and 5 mm for anchoring to the proximal side of the thrombus. To allow for an entire thrombus to be placed inside the suction cup, the length of the suction cup may be up to 50 mm. Preferably, the length of the suction cup is between 0.1 mm and 1 mm at least in a first shape adapted for navigation in a tortuous vascular network.

The suction cup allows attachment to the thrombus resulting from a depression inside the suction cup. This bond may be strong enough to allow the transfer of the pulling force and displacement created by pulling of the distal part of the catheter device via the flexible line onto the thrombus. The force may be sufficient to overcome other external forces acting on the thrombus such as forces caused by artery walls squeezing the thrombus, friction between the thrombus and the artery walls, irregularities on the arteries surfaces, proximal blood pressure acting on the thrombus, and/or blood flowing on and around the thrombus.

The efficacy of the suction cup may increase with pressure difference between the inside of the suction cup and the pressure acting outside of the suction cup. The efficacy may also increase with the effective surface on which the suction cup contacts the thrombus. To maximize radial compactness and effective suction surface, the suction cup may have a thin outer rim. The depression may be in a range between -1 bar and 0 bar. The inner diameter of the suction cup area, which substantially corresponds to the interface interacting with a thrombus when attached, may be in the range 0.5 mm and 3 mm. The thickness of the suction cup rim formed substantially by the suction cup wall edge may range between 50 um and 300 um.

The efficacy of the suction cup in attaching to a thrombus may depend in its stiffness. A flexible rim may be used to allow for adaptation of the rim to the thrombus and to provide a fluid-tight seal. Adaptation of the rim to the thrombus due to its mechanical flexibility may also prevent accidental detachment due to off-axis pulling.

The lumen of the flexible line may not be large enough for penetration of a thrombus on a long length. The lumen of the flexible line may be in the range of approximately 300 um. Consequently, the contact surface of the thrombus with the retrieving system may be less compared with an aspiration catheter, which may have as much as 2 mm in lumen diameter. The suction cup therefore provides optimized anchoring of a thrombus and therefore allows to use thinner structures which provide better access to tortuous systems.

The suction cup may comprise any one of the following features:
A suction cup lip may be used to provide improved radial distensibility that causes it to extend radially when pressed against a thrombus. This makes the suction cup more compliant to the thrombus geometry, improves the effective suction diameter and thus holding force while at the same time reducing the forces created by the blood pressure and flow on the thrombus. The suction cup lip may be made of a polymer or elastomer, in particular with a tensile strain value at 100% elongation between 0,05 MPa and 50 MPa and hardness values between shore A0 and D100, and/or
the suction cup may be configured to expand in the direction perpendicular to its compression due to the Poisson effect, and/or
the suction cup lip may comprise a mesh embedded in an elastomer, and/or
the suction cup lip may have a distribution of materials of different stiffness, and/or
the suction cup lip may have longitudinal stripes of materials of.different stiffness distributed around the suction cup's circumference, and/or
the suction cup lip may have longitudinal groove-like geometrical features distributed around the suction cup's circumference.

The suction cup lip may also be configured to radially retract and clamp/squeeze the thrombus when being pulled and thus adding extra holding force at the time of extraction, for example by using the Poisson effect.

In some embodiments, the suction cup lip may have auxetic properties, i.e. exhibit a negative Poisson's ratio and expand in a direction perpendicular to the direction in which it is stretched. For example, a suction cup may have a reduced shrunken diameter that anchors to the thrombus. When pulled back, it expands radially, pressing against the artery's wall. The depression is then stopped while the pulling force is maintained, causing the tip of the lip of the suction cup to open further. This pulling force is now acting due to the friction force of the artery's wall thus maintaining the suction cup expanded. The depression can then be brought back to anchor once against to the thrombus, this time with a greater effective diameter and suction force.

In some embodiments, the suction cup lip may have auxetic properties, i.e. exhibit a negative Poisson's ratio and expand in a first direction perpendicular to a second direction in which it is stretched.

This property may be produced through the use of a mesh structure made.

The mesh material may be elastic so as to enable the mesh deformation under load and subsequent recovery. For example, the suction cup may be made using nitinol wires arranged in a cylindrical mesh with an auxetic structure.

This mesh may be embedded inside a non-porous and soft polyurethane rubber sheath. The sheath's may be adapted to deform with the mesh while keeping the suction cup leak-tight. This mesh may comprise or consist of super-elastic nitinol wires with a diameter in the range of 25 um to 200 µm diameter and arranged in a re-entrant honeycomb structure. A re-entrant honeycomb structure may comprise or consists of a lattice of concave hexagons. Each hexagon may have two opposite and equal reflex internal angles (also called re-entrant angles) greater than 180°. The remaining four internal angles are equal with respect to each other and regular, meaning they are less than 180°. To achieve the super-elasticity inside the human body, the selected nitinol wire may to be tuned to have austenite finish transition temperature below 37°C, preferably around 22°C. ,

The non-porous sheathing of that mesh may comprise or consist of polyurethane with a hardness ranging from shore A0 and D100 and a thickness ranging from 50 um to 300 um. The Poisson's ratio of the structures and materials described herein may range from -3 to -0,5.

An embedded auxetic suction cup may also comprise or consist of multiple other mesh structures in various materials, or by perforating a thin sheet of core material with an auxetic pattern. Certain materials may also have intrinsic auxetic properties and may thus be used for single-material or composite auxetic suction cups as well as in methods manufacturing the same. Such materials include some biomaterials but also polymeric fibrous, microporous and molecular-level auxetic materials.

In an alternative embodiment, the suction cup lip has a positive Poisson's ratio such as to expand radially when axially pressed against a thrombus.

The depression in the suction cup may varied (e.g. by using a valve and/or pump control) such as to turn the vacuum on and off quickly in a cyclic fashion. This may improve the suction force and accelerate the thrombus. The depression may be varied from 0 bar to -0.99 bar, preferably from -0.5 to -0.95 bar (e.g. relative blood pressure or atmosphere). The frequency of the pressure change may be, e.g. 10 Hz.

The surface roughness, the stiffness and/or the surface area of the inner surface of the suction cup may be dimensioned such as to increase the friction between the suction cup and the thrombus and thus the holding force.

In particular, the roughness values (Ra) may be above 3.2 um. The roughness of the surface may trigger some coagulation reactions with the red blood cells on the surface of the thrombus and cause supplementary adhesion.

The inner surface of the suction cup may be covered with irregularities such as hook-like features that may attach to the thrombus, thus resulting in supplementary holding force.

Channels may allow for redirection of the depression to otherwise unreachable thrombus areas, thus increasing the overall holding force.

The inner surface of the suction cup may be configured to increase its temperature to heat the surface of the thrombus and thus creating cauterization. As a result, the thrombus may be attached more securely. The temperature may be increased up to 60°C, preferably to a temperature between 40 to 45°C.

The inner surface of the suction cup may be coated with a thrombogenic agent such as thrombin. The coagulation reaction may improve the anchoring with the thrombus. To avoid unwanted thrombogenic reactions, e.g. during navigation and/or before treatment, the suction cup may be covered. Preferably, the suction cup is coated with a layer of polymer such as polyurethane, poly(lactic-co-glycolic acid (PLGA), and/or polydioxanone (PDO). The layer may have a thickness between 50 um to 300 um and may be attached to the magnetic part and/or be biodegradable. The coating may be broken by flushing a solution, in particular a saline solution. Such a solution may be delivery via the flexible line.

The suction cup may be radiopaque. The suction cup may have different levels of radiopaque properties which may enable a detection of its orientation. For example, the distal part of the cup may have more contrast than the proximal part. For example, a specific radiopaque ribbon may be set on the distal part of the cup.

The suction cup may be expandable and thus to be deployed once at the thrombus site. Thus, in a delivery configuration, the suction cup may have a smaller cross section and/or size. Controlling the deployment of the suction cup may thus improve navigation, may protect the suction cup from premature clogging, may prevent backward blood flow through the device by closing its distal access, may allow the suction cup to adjust to the artery minimizing the effect of blood pressure and flow, may allow the suction cup to adjust to the thrombus diameter increasing the effective suction surface and potentially adding clamping force around the thrombus. Moreover, an expandable suction cup may maintain the artery in its larger systolic diameter. In such case, the suction cup deployment may be performed right before the end of the diastole.

The suction cup may be deployed and retracted using an adjustable and preferably reversible external constraint. The constraint may in particular comprise or consist of a spring-like suction cup with an external sheath that may be retracted or expanded on demand to deploy or retract the suction cup and/or a ring around a spring-like suction cup lip. Such a ring may be adjusted in diameter, for example by cable or heat actuation. Heat actuation may be done using a ring made of a shape memory alloy exhibiting one or two-way shape memory effect.

The suction cup may be deployed using a single-use external constraint. For example, a breakaway cap or a foil may be used, and/or a breakaway filament and/or mesh.

A breakaway cap or foil holding a spring-like suction cup shut may be used. Such a structure may be broken to release the suction cup, for example by adjusting the pressure inside the lumen of the flexible line or using a hydraulic flow or using heat or by pulling or with a magnetic force.

A breakaway filament or mesh holding a spring-like suction cup shut may be broken to release the suction cup, for example by Joule heating.

A spring-like suction cup, held shut by a depression during insertion and navigation, may be released once at the thrombus site. Once released the suction cup opens and the depression can be reapplied in proximity to the thrombus without the cup closing on itself again due to the presence of the thrombus and/or due to the structural integrity of the suction cup overcoming the closing force caused by the depression.

The suction cup, in particular the suction cup lip, may comprise or consist of a shape memory alloy (SMA), e.g. to control its deployment.

SMAs, such as nickel-titanium alloy (also known as Nitinol) may recover a given shape when heated above a transition temperature, typically the austenite transition temperature. This may be possible even if the part has been plastically deformed in its cold martensite structure. A typical value is up to 8% recoverable strain in the martensite phase. Both the shape and transition temperatures may be tuned.

Certain SMA may also exhibit a two-way shape memory effect which may enable a part made of SMA to switch between two "memorized" shapes: one in its austenite structure (hot) and another one in its martensite structure (cold). This can be done externally (extrinsic two-way effect) by adding constraint to the SMA part for example with springs or internally (intrinsic two-way effect) by introducing permanent internal stress in the SMA. The intrinsic two-way effect can for example be implemented through a process called "training" which often entails severe plastic deformation.

SMAs may also exhibit pseudoelastic properties which allow SMA parts in their austenitic phase (hot) to recover large strain with little to no permanent deformation. These strains may be greater than 10%.

SMA may be implemented in the suction cup design is several ways. For example, a suction cup lip made of a SMA mesh may be embedded inside an elastomer, one or several SMA flat spring may be embedded inside an elastomer, and/or a SMA torsion spring may be embedded inside an elastomer.

A suction cup made of an SMA may be trained to memorize a cylindrical austenite shape at a large, set diameter (for example 5 mm). When actuated, this allows it to expand to the entire diameter of smaller arteries (shape memory effect) and conform to its internal wall surface (pseudoelasticity) while maintaining a radial outward mechanical constraint preventing it from collapsing on itself or on the thrombus when applying the depression. This design may allow maximizing a suction diameter and thus suction force, while removing all or almost all adverse forces caused by blood pressure and blood flow.

To this end, the SMA suction cup may be in its austenitic phase at body temperature. In order to prevent early deployment and guarantee navigability when going towards or away of the thrombus site, several options are conceivable.

For example, the SMA suction cup may be compressed and may navigate inside the flexible line and may be pushed out once at the thrombus site.

Additionally or alternatively, the SMA suction cup may be compressed and may navigate inside a sheath which may be pulled or pushed on demand to deploy or retract the suction cup. Once anchored to the thrombus, pushing the sheath may help retract the suction cup and thus may create an extra holding force by radial clamping of the thrombus and frees the artery thus minimizing damage to the arteries wall when pulling.

Additionally or alternatively, the SMA suction cup may be compressed with an adjustable ring around a suction cup lip. The suction cup lip may be heated (e.g. if made itself of a SMA) or cable actuated.

Additionally or alternatively, the SMA suction cup may be compressed inside a breakaway sheath or foil that may be broken. For example, breakage may be achieved by adjusting the pressure/depression inside the medical device or having a hydraulic flow, and/or using heat and/or by pulling and/or with a magnetic force.

Additionally or alternatively, the SMA suction cup may be held compressed by a mesh of filament that may be broken, for example using joule or induction heating.

Additionally or alternatively, the SMA suction cup may be held compressed by a depression until its released.

Additionally or alternatively, the SMA suction cup may be tuned or thermally insulated in such a way that a thermal resistance causes the time delay until it reaches body temperature. As a result, the deployment may be longer than the navigation time to the thrombus site.

Additionally or alternatively, the SMA may be held at a lower temperature than its austenitic transition temperature, for example by using a thermal insulation by a material which may be mechanically, electrically, biologically and/or thermally degraded once on site.

Additionally or alternatively, the SMA suction cup may have an extrinsic or intrinsic two-way memory shape effect. Once the thrombus is sucked in, the suction cup may retract by cooling, allowing to create extra holding force by radial clamping of the thrombus and freeing the artery thus minimizing damage to the arteries wall when pulling. The cooling may be produced, for example, by using a thermoelectric effect such as Pelletier effect or Thomson effect or the thermodynamic Joule-Thomson effect. Additionally or alternatively, a cold saline solution may be injected to cool the suction cup. It will be understood that cool, in this context, may be understood as a temperature lower than the martensite transition temperature and may for example, depending on the SMA used, be in the range of 15°C to 35°C.

Additionally or alternatively, the SMA suction cup may have an extrinsic or intrinsic two-way memory shape effect. Once the thrombus is sucked in, the suction cup may retract by cooling, allowing to create extra holding force by radial clamping of the thrombus and potentially freeing the artery's wall thus minimizing damage to the arteries wall when pulling.

The cooling may be produced, for example, by using a thermoelectric effect such as Peltier effect, Thomson effect, the thermodynamic Joule-Thomson effect, and/or by injecting cold saline solution. The cold saline solution may be at a temperature below 37°C, preferably between 4°C and 35°C.

The suction cup may be made of a cylindrical nitinol wire mesh embedded inside a non-porous polyurethane rubber sheath. The sheath's purpose is to follow the mesh's deformation while keeping the suction cup leak-tight. This mesh could use nitinol wires ranging from 25 um to 200 µm diameter and arranged in a specifically-tuned structure to reach the desired expansion force and radial distensibility. This may be, for example, a honeycomb or diamond-shaped structure.

A honeycomb structure may comprise or consist of a lattice of convex hexagonal cells, meaning each internal angle is equal and less than 180°. The hexagonal cells may be regular, meaning each internal angle is equal, or irregular, meaning each internal angle can be different but still less than 180°.

A diamond-shaped structure may comprise or consist of a lattice of rhombus-shaped cells.

The suction cup may work in a similar way as a as known from vessel stents, wherein the structure is adapted to radially expand once at a desired location (using a specifically designed and tuned nitinol mesh). The suction cup may, in addition, have a non-porous elastic sheathing for sealing.,

The nitinol mesh may be trained to memorize an austenitic cylindrical shape ranging from 0 mm to 50 mm in outer diameter, preferentially 1.5 mm to 5 mm. This diameter may be greater than that of an artery, but typically the mesh may be designed so as not to exert a significant force on the artery walls so as to avoid damage or burst of the artery. If properly designed, the expanded cylindrical nitinol mesh may be retained by the artery and, as a result, the expanded suction cup may have an effective diameter which is maximized in view of the artery size and in consideration of its distensibility.

The non-porous sheathing of the mesh may comprise or consist of polyurethane with a hardness ranging from shore A0 and D100 and a thickness ranging from 50 um to 300 um. The mesh structure may favor radial expansion when the nitinol is in its austenitic super-elastic state. This may, for example, be a honeycomb or diamond-shaped structure.

To achieve super-elasticity inside the human body, the selected nitinol wire needs to be tuned to have an austenite finish transition temperature below 37°C, preferably around 22°C. However, it is possible to use any austenite finish transition temperature between 22 and 37°C to tune the time delay after which expansion of the suction cup begins when inserted inside the body and navigated to the thrombus site. To achieve the suction cup retraction, the selected nitinol wire needs to be tuned to have a martensite start transition temperature below 37°C, preferentially between 4 and 20°C.

A two-way shape memory effect which may cause the constriction of the suction cup in its martensitic state may be produced intrinsically. This includes inducing internal stresses in the Nitinol alloy that favour certain martensitic shapes. This may be done through various treatments such as plastic deformation in martensite, super-elastic training, stress-assisted ageing and stress-induced martensite ageing.

This two-way effect may also be produced extrinsically, which means that a external force acting on the Nitinol alloy may cause the shape change. For example, a polymer sheath, e.g. made of a polyurethane, may be moulded and hardened on the trained cylindrical nitinol wire mesh in its martensitic state. When the nitinol mesh is brought in its austenitic state and expands, the sheath is stretched due to the shape change of the Nitinol mesh, and create an counter force. This force is such that when the nitinol mesh is cooled back in is martensitic mesh, it becomes unable to resist the sheath's force and retracts.

Proper tuning of the force exerted by the polyurethane rubber sheath may be required. Typically, a counter force is tuned so as to cause the martensitic wire mesh constriction, yet the counter force may be small enough to allow the austenitic wire mesh expansion. This tuning may be done by selecting the thickness and hardness of the sheath accordingly to the dimensions, shape and training of the nitinol wire mesh.

An extrinsic two-way effect, where the cup retracts in its martensitic state, may also be produced by using springs opposing the cylindrical nitinol mesh's austenitic expansion. For example, radially placed flat springs or torsion springs may be used and comprise or consist of nitinol. These nitinol springs may be maintained in there austenitic super-elastic state under ambient and/or physiological conditions. Thus, their austenite finish transition temperature may be lower than the martensite start transition temperature and the austenite finish transition temperature of the nitinol wire mesh, and preferably lower than the martensite finish transition temperature the austenite start transition temperature of the nitinol wire mesh. The nitinol springs austenite finish transition temperature may be lower than 37°C.

In one configuration, the austenite finish transition temperature is preferably lower than 22°C and may, in particular, match the austenite finish transition temperature of the nitinol wire mesh.

In another configuration, the austenite finish transition temperature is preferably lower than 18°C and may ensure that the nitinol springs stay in their austenitic state at room temperature. Nitinol flat springs have a rectangular cross section, with a thickness ranging from 25 um to 200 um and a width ranging from 100 to 500 um. A length of the flat spring may range from 1 mm to 150 mm depending on their layout. The width of the spring may refer to a dimension which is arranged radially relative to the cylindrical nitinol mesh and may, in particular, be a smallest dimension. The length and width may be dimensions in a tangential direction relative to the cylindrical nitinol mesh and may, in particular, be the largest and second-largest dimension, respectively. These flat spring may be bent and laid against the cylindrical nitinol mesh, for example in a circumferential orientation. Flat springs may be laid in several shapes. The flat spring may be laid in a ring shape such that its length-wise neutral axis is in a orthogonal section of the cylinder. In another configuration, the flat spring may be laid such that its length-wise neutral axis in in the same plane as the cylinder's axis. In another configuration, the flat spring may be laid helically such that its length-wise neutral axis forms a helix around the cylinder's axis. The helix' radius may be uniform or variable. Several flat springs may be placed around the same cylindrical nitinol mesh. For example, if laid in a ring shape, two or three springs may be placed in parallel orthogonal sections of the cylinder. These various springs configuration may be combined in various shapes and dimensions so as to shape the initial cylinder mesh in a different geometry.

It is also conceivable to induce the shape change by heating instead of actively cooling. Heating may be done, for example, using joule heating, induction heating, or by injecting hot saline solution. The hot saline solution may be at a temperature above 37°C and preferably lower than 45°C. In this case, the trained cylindrical nitinol wire mesh may act as a constricting element in the austenitic state could be trained to memorize an austenitic cylindrical shape ranging from 0.2 to 2.5 mm in outer diameter. The austenite finish transition temperature may be above 37°C so as to not induce a (complete) phase change at body temperature. Preferably, the austenite finish temperature is above 43°C to take into account a potential increase of body temperature caused by fever.

Radially placed flat springs or torsion springs may maintain the cylindrical nitinol mesh in a larger configuration, i.e. with a larger outer diameter. The outer diameter may be between 1 mm and 50 mm, preferably between 1 mm and 3 mm. In general, the outer diameter may be chosen to be smaller than the artery's inner diameter to enable navigation of the suction cup to the thrombus site in its expanded state.

Additionally or alternatively, the outer diameter may be between 1 mm and 10 mm in its expanded state, preferably between 1.5 and 4 mm and in particular larger than the artery's inner diameter so as to enable its expansion up to the targeted artery's wall. In this configuration, the navigation of the suction cup to the thrombus site may be done in its constricted state.

The springs may be made of nitinol. Nitinol springs may be maintained in there austenitic super-elastic state throughout any use and/or treatment. Therefore, the austenite finish transition temperature of the springs may be lower than the martensite start transition temperature and the austenite finish transition temperature of the nitinol wire mesh, preferably lower than the martensite finish transition temperature and/or the austenite start transition temperature of the nitinol wire mesh. The nitinol spring's austenite finish transition temperature may be lower than 37°C, preferably lower than 18°C, so as to make sure the nitinol springs stay in their austenitic state even at room temperature. The nitinol springs may, in particular, have any of the properties of the nitinol flat springs described above, for example theirsizes, shapes and arrangements.

Another possibility to implement a two-way shape memory effect in a suction cup may be a suction cup with two opposing cylindrical nitinol wire meshes embedded inside a non-porous polyurethane sheath. Such a suction cup may be referred to as a "composite suction cup". For example, a first wire mesh may be trained to have a small cylindrical shape having an outer diameter in the range from 0.2 mm to 25 mm in its austenitic state and may act as a constricting wire mesh. A second wire mesh may be trained to have a cylindrical shape with a larger outer diameter ranging from 0 mm to 50 mm, preferably between 1 mm and 5 mm and may act as an expanding wire mesh. Thus, the shape change induced by the first wire mesh constricts both the first and the second mesh. When the second mesh is expanded, the first mesh is also expanded. In this case, both the first and the second mesh may need to have different transition temperatures. The austenite finish transition temperature of one mesh needs to be lower than the martensite start transition temperature and the austenite finish transition temperature of the other mesh, preferably lower than the martensite finish transition temperature and the austenite start transition temperature of the other mesh. The mesh with the higher austenite finish transition temperature needs to exert a higher constriction or expansion force than the opposite force exerted by the mesh of lower austenite finish transition temperature. For example, these forces can be tuned by training each mesh to a specific austenitic diameter before assembly.

As such, preferably, one mesh is in its austenitic state, and has superelasitic properties at an ambient temperature, while the respective other mesh is in a martensitic state at the same ambient temperature. By changing from martensitic to austenitic through temperature actuation, the second mesh may perform a shape change.

Alternatively, both meshes may initially be martensitic at ambient temperature. By heating, the first mesh reaches its austenitic state, causing either constriction of expansion. By heating to a higher temperature, the second mesh is then actuated, also reaching its austenitic state and moving the cup in the opposite direction of what the first mesh caused.

In one configuration, the composite suction cup may be tuned to be actuated by active heating. In this configuration, the austenite finish transition temperature of either the first or the second mesh may be at a temperature above 37°C, preferably above 43°C so as to take into account a potential increase of body temperature caused by fever. The respective other mesh may have an austenite finish transition temperature is below 37°C, preferably around 22°C.

In another configuration, the composite suction cup may be tuned to be actuated by active cooling. In this configuration, the austenite finish transition temperature of the first or the second mesh may be below 37°C, preferably around 22°C. The respective other mesh may have an austenite finish transition temperature below 22°C, preferably below 18°C.

Additionally or alternatively, the SMA suction cup may have a positive Poisson's ratio tuned in such a way that pulling on it while it is anchored to the thrombus causes it to retract radially, allowing to create extra holding force by radial clamping of the thrombus and freeing the artery thus minimizing damage to the arteries wall when pulling.

For example, the suction cup may comprise a super-elastic mesh made of nitinol wires with a wire thickness ranging from 25 um to 200 um diameter and arranged in a structure that has a positive Poisson's ratio. Such structures may include honeycomb or diamond-shaped structures. To achieve the super-elasticity inside the human body, the selected nitinol wires may have austenite finish transition temperature below 37°C, preferably around 22°C. A non-porous sheathing may be arranged with respect to the mesh. The sheath may comprise or consist of polyurethane with a hardness ranging from shore A0 and D100 and a thickness ranging from 50 um to 300 um. Typical values of Poisson's ratio for suitable structure may range from 0.5 to 3.

In certain embodiments, the SMA suction cup may also be at least partially in its martensitic phase at body temperature. In order to allow deployment once at the thrombus site and retraction before pulling, several options are conceivable.

For example, the SMA suction cup may be deformed and compressed in its martensitic phase before entering the patient's body. To avoid unwanted deformation during navigation, it can be confined inside the flexible line or inside a sheath or a foil or any other encapsulating body from with it can be freed by manual pushing, manual pulling and/or by breaking when expanded in its austenitic phase.

Once released, e.g. at a thrombus site, the SMA suction cup may be expanded by heating to its austenitic transition temperature. Heating may be performed by, e.g., Joule heating, induction heating, chemical heating or induction of a warm biological liquid. For improved safety, the austenitic transition temperature may be close to the body normal temperature, for example between 38.5°C and 43°C. This may also limit the energy needed for heating. Once the suction cup is anchored to the thrombus, it is left to cool below its martensitic transition temperature. The suction cup then may retract around the thrombus due to the depression on its inside and thus freeing the artery's wall. The cooling may also be sped up for example by using thermoelectric effect such as Pelletier effect or Thomson effect or the thermodynamic Joule-Thomson effect.

Additionally or alternatively, the SMA suction cup may have an extrinsic or intrinsic two-way memory shape effect causing it to have a retracted stable martensitic phase. This reduces the likelihood of the suction cup deforming during navigation in its martensitic phase. This may also increase the clamping force around the thrombus during retrieving.

The SMA suction cup may be trained to memorize a cylindrical austenite shape at a small set diameter (for example 0,5 mm). When actuated, this allows it to retract (shape memory effect) and compress against the thrombus while conforming to its surface (pseudoelasticity), adding clamping force to the suction force. To this end, an extrinsic or intrinsic two-way shape memory effect with the martensitic phase or the austenitic phase at body temperature may be used.

It will be understood that any combination of the above concepts related to SMAs may be used.

The flexible line is compatible with the magneto-fluidic navigation. Magneto-fluidic navigation may refer to the advancement of a device, here in particular the distal part of the catheter device, by being carried in a fluid flow and pushed by a drag force and being guided and/or steered by a magnetic force. In the case of the magneto-fluidic navigation, the displacement of the distal part is not substantially influenced by a pushing force exerted via other parts of the device, because the rigidity of the flexible line is not sufficient to transfer such a translation movement.

Preferably, the bending stiffness of the flexible line is low enough such that a force exerted on the magnetic part attached thereto is smaller than the magnetic force exerted by the external magnetic field, or a drag force exerted by blood flow. In particular, a bending stiffness of the flexible line may be in the range of 1 mN•mm² to 100 mN•mm².

The flexible line may comprise a radiopaque material for visualisation of the flexible line. This may facilitate the monitoring of the flexible line during navigation and could contribute to an improved initial positioning of the attachment element with regards to the thrombus. In turn, this may help streamline the procedure and reduce the risk of complications linked to twisting or kinking of the flexible line as this would be detected early.

The radiopaque material may comprise any one or more of: a metal; a radiopaque compound. Particularly, the radiopaque material may comprise one or more of: barium sulfate; bismuth; bismuth subcarbonate; bismuth trioxide; tungsten; platinum; gold; silver; iodine; tantalum.

It is conceivable that radiopaque material may be provided as a coating to the flexible line. Additionally or alternatively, the radiopaque material may be provided as discrete elements along the flexible line. The radioopaque element may be placed inside the flexible line or on its surface.

In some embodiments the flexible line may comprise at least one reinforcement. The reinforcement may extend along at least part of the flexible line. Alternatively, a plurality of reinforcement may be provided along at least part of the length of the flexible line. The reinforcement may be configured to prevent the flexible line of the device from deforming and/or kinking during navigation. The reinforcement may be configured to reinforce the flexible line at its position. For instance, the reinforcement may increase the stiffness of the flexible line at its position. This allows the flexible line to be flexible enough to be easily navigated through the vessel to the thrombus whilst also having the stiffness necessary to avoid twisting and kinking of the flexible line.

Optionally, a plurality of reinforcements may be provided along the flexible line. The plurality of reinforcements may be at least partly distanced from one another. The plurality of reinforcements may provide the flexible line with varying degrees of stiffness.

It is conceivable, that at least one reinforcements may be any one or more of: a longitudinal reinforcement; a circumferential reinforcement; helical reinforcement; a lattice reinforcement. The reinforcement may be shaped as any one or more of: a ring; a coil; a ribbon; a grid; a lattice; a tube; a rod; ribbon.

The reinforcement may be embedded in the body of the flexible line. Additionally or alternatively, the reinforcement may be layered on the outer surface of the flexible line. Further additionally or alternatively, the reinforcement maybe provided as a layer on the inner surface of the flexible line.

In some embodiments, the reinforcement may be configured to allow visibility of the flexible line as it is navigated within the patient. To achieve this, at least a portion of the reinforcement may comprise a radiopaque material. Preferably, the reinforcement may comprise sections of radiopaque material. These sections of radiopaque material can optionally be spaced by sections of non-radiopaque material.

Proximal attachment of the device at a thrombus is particularly advantageous because it enables a quick attachment without the need to penetrate or navigate device elements in or around the thrombus. In addition, because a pulling force is exerted on the proximal face of the thrombus, the thrombus may be stretched, which may lead to a smaller diameter, thus reducing the necessary pulling force, as the friction between the vessel wall and the thrombus may be reduced.

A proximal face of the thrombus may be understood as the proximal-most area of the thrombus in a direction towards the device, i.e. in a direction of intended removal of the thrombus.

The suction mechanism generally comprises an opening in the suction cup and a suction line which particularly preferably extends through the flexible line. The suction line may be connected to a vacuum pump, in particular via a connector.

The combination of a main body and a distal part connected to the main body by the flexible line is particularly advantageous to provide a fast and safe treatment. The flexible line is mechanically more flexible than the main body of the catheter, which thus may serve as a rigid element for pushing through a vasculature. Therefore, it is possible to move though larger vessel portions, for example, relatively quickly by pushing the catheter main body. When reaching a treatment site however, it may be necessary to reach smaller structures and/or orient the attachment element more precisely to attach to the thrombus. This is enabled by releasing a guidable attachment element which is attached by a flexible line.

The suction cup may have a generally flat shape with a rim, a curved shape, and/or comprise peripheral protrusions to provide sufficient fit with a shape of the thrombus.

An inner diameter of the suction line may be in the range of 50 um to 800 µm, preferably 200 um to 500 um. The suction line may be formed by a channel inside the flexible line or as a separate line (i.e. having a suction line wall) arranged inside the suction line, or elsewhere with respect to the suction line.

It will also be understood that the suction line may additionally be used for other purposes such as delivery of a gas and/or liquid, in particular drug-containing fluids in certain embodiments.

It is therefore conceivable that the catheter device may comprise or may be connectable to a container configured for holding the gas and/or liquid. When provided or connected, the container is in fluid communication with the suction line.

Additionally or alternatively, the suction line may serve to deliver a biocompatible liquid agent within the occluded vessel. The liquid agent may be a wetting agent and/or a lubricant for reducing friction between the thrombus and the epithelium of the vessel.

Additionally or alternatively, the catheter device may comprise an outer specific connection for pushing the liquid (e.g. a lubricant liquid) into the suction line. For example, a syringe pump may be used to delivery the liquid. The outer specific connection may be a tubing connected to the proximal side of the device, preferably via a two-way or three-way valve. The outer specific connection may be a transparent tubing (for example made of Tygon^{®}). Syringe pumps are particularly accurate.

The wetting agent may comprise a non-ionic surfactant. The non-ionic surfactant could include any one or more of a glycerol-based solution; a sorbitol compound or a derivative thereof; an ethoxylated fatted alcohol or derivates therefor; an Ethoxylated sorbitan ester or a derivative thereof; a poloxamers or derivatives thereof. Thus, the wetting agent may comprise any one or more of Polysorbate 80 (Tween 80); Sorbitan Monooleate (Span 80); Polyoxyethylene(23)lauryl ether (Brij 35); Poloxamer 124; Poloxamer 188; Poloxamer 407; Poloxamer 388; Poloxamer 188/182.

The biocompatible lubricant may include any one or more of a glycerol-based lubricant; a heparin-based lubricant; a silicon-based lubricant; a polyurethane-based lubricant; hydrogel-based lubricant; a polymer-based lubricant; a poloxamer-based lubricant; a water-based lubricant; an oil-based lubricant.

The provision of the lubricant may facilitate the extraction of the thrombus by reducing the friction between the epithelium of the vessel and the thrombus when the thrombus is extracted. This in turn reduces the risk of the thrombus detaching from the attachment element and may improve the duration of the intervention by avoiding the necessity of repositioning the device to reattach the thrombus.

The Young's modulus of the flexible line and/or the suction line 3 (in particular if configured as a separate element) may be in the range of 0.001 GPa and 100 GPa, preferably 0.001 GPa and 5 GPa, in order to be compatible with navigation within a vessel system.

In some embodiments, the device comprises more than one attachment element, for example two attachment elements or three attachment elements. Multiple attachment elements can reduce the mechanical stress on a thrombus during retrieval and thus make the treatment safer.

Multiple suction cups may be present, for example three suction cups.

The catheter device may comprise one or several balloons arranged at a distal portion of the catheter main body or on the distal part of the catheter device.

A balloon used in combination with the device may in particular be inflatable asymmetrically. For example, the balloon may be selectively inflatable only on one side with respect to a longitudinal axis. Alternatively, several selectively inflatable balloons may be used. This may allow to detach the device from a tissue to which it has been attached deliberately or by accident.

The thickness of the balloon may be between 50 um and 300 µm, preferably 80 µm and 150 um. Furthermore, an activation system for inflating or enabling inflation may be present. The balloon may have a spherical shape with a diameter between 50 um and 700 µm, preferably between 200 µm and 400 um. Thus, the contact surface with a tissue wall (and thus inadvertent adhesion) may be reduced. The balloon wall may be made of any suitable, medi-, cal-grade polymer, such as polyurethane and/or silicone. The activation system may comprise or consist of a solenoid valve and/or have a shape corresponding to a solid of revolution.

In a preferred embodiment, the catheter main body may have a mechanism to release at least the attachment element from a storage area of the catheter main body. To this end, the catheter main body may comprise, at a distal end, a closing mechanism such as a diaphragm. The closing element may isolate the attachment element while navigation and may be selectively opened to release the attachment element for treatment. After treatment, the thrombus may be pulled back may keep a thrombus inside the catheter. When the closing mechanism is opened, its opening may have a diameter may be between 1 mm and 4 mm, preferably between 2 mm and 3 mm.

The outer diameter of the catheter main body may be between 0.8 mm and 5 mm, preferably between 1 mm and 2 mm.

It is conceivable to use more than one suction line. Additional hollow lines may be used to activate additional features of the catheter device, and of the attachment element in particular, for example for the injection of saline solution or the inflation of a balloon.

The attachment element may additionally comprise any element adapted to attach to a thrombus, in particular it may comprise structure for mechanical interaction such as forks, but also glues, fibers. The attachment element may further be connected to the magnetic part, in particular through a form-fit connection.

In some embodiments, an electric element, preferably a ring, is arranged with respect to the attachment element such that an electric current may be applied to a thrombus after it has been sucked by the suction mechanism.

Preferably, the catheter device comprises an activation mechanism. For example, the attachment element may have an activated state and a deactivated state. In the deactivated state, the attachment element is adapted to not interact with a vessel wall or with the thrombus. In the activated state, the attachment element is adapted to interact with the thrombus. The activation mechanism is adapted to bring the attachment element at least from the deactivated state to the activated state.

An activation mechanism may be advantageous because inadvertent interaction between the attachment element and tissue and/or blood can be prevented during delivery, which may lead to safer treatments and less complications, and easier delivery.

In general, the medical device may be moved backward to a delivery catheter once the thrombus is deblocked from the artery.

Once in the delivery catheter, the medical device may be moved backward through the delivery system. Then the access valve with the delivery catheter may be closed and the vacuum may be stopped. The thrombus may then be collected.

Additionally or alternatively, a retrieving catheter may be used. To this end, the invention is further directed to a kit comprising a retrieving catheter and a catheter device as described above. The retrieving catheter and the catheter device may be combined with a delivery catheter through which both the retrieving catheter and the catheter device may be delivered.

A retrieving catheter is particularly advantageous when the distal part of a device is occluded by the thrombus or a portion of the thrombus. Then, the device may need to be removed from a delivery catheter. This is time consuming and may increase the time for successful recanalization. Hence, a retrieving catheter may reduce the time required to extract the thrombus from the vascular network.

In some embodiments, the retrieving catheter is formed by the delivery catheter or formed as separate catheter to be intro-duced via the delivery catheter.

The retrieving catheter may have no closing element. Once the medical device according to the invention is inside the delivery catheter, preferentially with the distal part near the distal part of the delivery channel, the vacuum is applied in the retrieving channel. Then, the vacuum is stopped in the medical device. To facilitate the extraction, a solution may be injected through the medical device.

The retrieving catheter may comprise a distal closing system.

When the retrieving catheter is formed as part of the delivery catheter, the delivery catheter may comprise a dual lumen, wherein a first lumen is adapted for the delivery of the medical device, which may be a delivery channel, and a second lumen is adapted for the thrombus retrieving.

The delivery channel may protect the medical device from a significant bending during the extraction of the thrombus by suction. Once the distal part of the medical device is at the border of the delivery channel, the closing element of the retrieving catheter may be closed. Hence, the lumen of the retrieving catheter is closed. Then the vacuum in the medical device may be closed and its lumen may be flushed with a solution, for example a saline solution or a radiopaque solution.

The flush may lead to the detachment of the thrombus from the suction cup. Then, a vacuum may be applied in the retrieving channel leading to the aspiration of the thrombus. Once the thrombus is removed from the lumen of the retrieving channel, the closing element may be opened leading to the opening of the lumen of the retrieving catheter. Then, the medical device may be deployed for a further treatment.

The retrieving catheter may have an inner diameter between 2 mm and 6 mm, preferably between 3 and 4 mm.

The retrieving catheter may be adapted for providing an aspiration of the thrombus by an aspiration catheter setup in the retrieving lumen. This may ensure a smooth extraction of the thrombus along the retrieving channel.

In some embodiments the retrieving catheter is provided as a separate catheter configured to at least partly house the catheter device. The retrieving catheter is configured to house the thrombus and medical device during extraction from the patient. Thus, the medical device may be retracted within the retrieving catheter after attaching to the thrombus via the attachment element. The retrieving catheter may be configured to retain the thrombus in its lumen. To this end a suction force may be provided within the lumen of the retrieving catheter. The lumen of the retrieving catheter may be connected to a vacuum pump, in particular via a connector and/or an access valve.

The retrieving catheter may therefore prevent the thrombus from re-entering the patient's blood .vessel if the thrombus detaches from the attachment elements during the retraction.

The vacuum pump to which the retrieving catheter and the medical device may be the same vacuum pump. Alternatively, the retrieving catheter and the medical device may be connected to separate vacuum pumps.

Additionally or alternatively, the retrieving catheter may allow for the passage of a balloon catheter. A balloon catheter may be inflated by the injection of a solution, for example a saline solution or a radiopaque saline solution. The balloon catheter may be used to decrease the blood flow velocity, in particular during the navigation and /or the retrieving step. The balloon catheter deployment may be controlled manually and/or by the magnetic guidance system.

In some embodiments, the flexible line of the device may comprise the balloon. Additionally or alternatively, the catheter main body may comprise a balloon.

Similarly to above, the balloon may be configured to reduce the pressure applied to the thrombus by the blood flow. This is achieved by inflation of the balloon within the vessel, thus reducing the flow of blood surrounding the thrombus.

The balloon may be inflated by injection of a solution, for example a saline solution. Alternatively, the solution may be a contrast solution, for example a radiopaque solution. Filling the balloon with a radiopaque solution allows the operator to visualize the position of the balloon and confirm the correct positioning of the device. The radiopaque solution may comprise any one of: an iodine-based solution, a Barium-based solution; a saline-based solution. Preferably, the radiopaque solution comprises one or more of barium sulfate; bismuth; bismuth subcarbonate; bismuth trioxide; tungsten; platinum; gold; silver; iodine; tantalum.

In some embodiments, the medical device is adapted for attaching or positioning, via a suction, a second medical device such as an aspiration catheter and move it.

This may provide for particularly easy and safe navigation of an aspiration catheter in cases where the use of an aspiration catheter is required. It is also conceivable to attach and move a microcatheter via the medical device.

To this end, the medical device may be attached, by suction, to a thrombus, may create a guide for an aspiration catheter up or near to a proximal surface of the thrombus. In this configuration, the medical device may be navigated up to the thrombus. Preferably, the outer diameter of the suction cup is between 1 and 2 mm. The distal part of the medical device may be attached to the proximal part of the thrombus. The rigidity of the flexible line may be increased by inserting a wire inside the flexible line. The wire may be made of polymer or metals or a combination. Preferably, the wire comprises a coiled wire arranged on a central wire, i.e. a structure like a guidewire. A coating, preferably a hydrophilic coating, may be arranged on the wire to improve its introduction in the flexible line. The aspiration catheter may be progressively pushed up to the thrombus following the flexible line. A distal part of the aspiration catheter may be mechanically flexible to enable its orientation toward the proximal surface of the clot. The medical device may be removed before starting the vacuum in the aspiration catheter.

The distal part of the aspiration catheter may allow for improved orientation. For example, the distal part of the aspiration catheter may comprise a folding structure which may be easily turned. A folding structure made of polymers, e.g. elastomers such as silicone and/or polyurethane, may be used. Blends and/or copolymers may also be used. Preferably, the folding structure has at least of three folds.

It is also conceivable to configure the folding structure as a portion of the aspiration catheter with reduced thickness. For example, the outer tube diameter of the aspiration catheter may be 2 mm with an inner diameter of 1.7 mm. The flexible distal portion of the aspiration catheter may be a tube with an outer diameter of 2 mm and an inner diameter of 1.8 mm. The flexible area has a length between 0.5 mm to 30 mm, preferentially between 1 to 5 mm.

The medical device may also be adapted to, in some configurations, be temporarily attached by suction on an artery wall to help the advance an aspiration catheter. Subsequently, the medical device may be released again to advance further. In in very tortuous networks, e.g. in succession of tortuous arteries, such an approach may be advantageous to reach a treatment site.

The flexible line of the medical device may be tensioned by anchoring the distal part of the medical device. The distal anchoring may be done by a magnetic force, a suction force or a combination of these forces. The anchoring may be done on a vessel wall or a thrombus. It may be possible to anchor the medical device several times during a navigation.

This allows for reducing or avoiding displacement of the flexible line due to different rigidities of the flexible line and the catheter which is attached to the medical device.

Additionally or alternatively, the rigidity of the flexible line may be increased by arranging a rod, e.g. a metal rod such as a NiTi rod, through the flexible line. The rod may be insertable temporarily, i.e. during a procedure, to temporarily increase the rigidity. The rod may have a diameter between 400 um and 100 µm, preferably between 250 µm and 150 um. A guidewire with a diameter between 400 um and 100 µm, preferably between 300 um and 200 um.

A preferred activation element may be a protective layer which may, for example, dissolve once exposed to blood.

The flexible line may be adapted to pull back a thrombus. In particular, it may be adapted, by material choice and/or appropriate dimensions, to have a minimum strength in the range of 0.1 N to 20N, preferably 8 N to 12 N.

The outer diameter of the flexible line may be between 100 µm and 1.5 mm, preferably between 500 µm and 700 um.

The flexible line may comprise or consist of a polymer, for example polyamide, polyurethane or elastomer such as silicone or a silicone-based material with a moderate breaking strain (lower than 100%) or a low breaking strain (lower than 10%) may also be used.

For example, the flexible line may substantially consist of a hollow tube made of silicone, having a high flexibility and a breaking strain of approximately 300%.

The holding line may be fixed to the magnetic part by glue, a knot, glue and resin, or a combination of different assembly methods which are known in the art.

The distal part of the catheter device, in particular the attachment element, may further comprise a sensor.

Additionally or alternatively the proximal part of the catheter device may comprise a sensor. Preferably the sensor is arranged between the vacuum and the main body of the catheter device. Additionally or alternatively a sensor may be provided within the vacuum pump.

Preferably, the sensor is adapted to determine incorrect or insufficient attachment of the attachment element to the thrombus.

It is conceivable that a plurality of sensors may be provided. The plurality of sensor may work together for enhance sensitivity of the monitoring of the attachment of the catheter device with the thrombus.

The sensor is preferably selected from the group comprising a force sensor, a temperature sensor, a pH sensor, an attachment sensor, a flow sensor, a pressure sensor, and a contact surface sensor.

For example, the sensor may comprise force sensor adapted to measure a pulling force acting on the thrombus and/or a sensor adapted to measure the contact surface between the attachment element and the thrombus.

The force sensor may measure the stretching of the flexible line during the retrieving of the thrombus. The force sensor may be set at the distal and/or at the proximal part of the flexible line. The values provided by the sensor may be used to check if the suction cup is anchored to the thrombus..In this case, during the proximal pulling, the flexible line is stretched. At the beginning of the retrieving, the flexible line is stretched and the distal part of the medical device is attached to the thrombus. The non-displacement of the distal part may be monitored under imaging. Once the thrombus is unblocked, the stress and/or the strain level in the flexible line may decrease. During the extraction step, if the force applied on the flexible line increases, this may indicate that the distal part of the medical device is encountering friction and / or blockage.

In particular, a pressure sensor may be used to monitor whether or not the attachment element is in contact with the thrombus and/or evaluate the nature of the thrombus.

Additionally or alternatively, the sensor may be adapted to monitor whether the attachment element is sufficiently attached to the thrombus during retrieval and or the extraction.

Thus, the catheter device may comprise any one or more of a force sensor; an electrical sensor; a flow sensor; a vacuum sensor.

A flow sensor may be used to monitor the flow of a fluid through the device. The flow may be monitored before and during the attaching and extraction of the thrombus to ensure the thrombus is sufficiently attached. For instance, the flow sensor will register a progressively reducing flow once the thrombus is attached to the suction cup. Continued monitoring during the extraction will show any increase of the flow rate. An increase of the flow rate might indicate a displacement of the clot in the suction cup.

A vacuum sensor may be provided to monitor the level of vacuum of the device when the thrombus is attached. The vacuum sensor registers the level of vacuum which can then be assessed as sufficient for extraction or not. Furthermore, a change in vacuum level could indicate the displacement or detaching of the thrombus from the suction cup, and thus indicate the need for reattachment or increased suction.

In some embodiments the catheter device may connect to a syringe configured to monitor the presence of blood within the catheter device. When and if provided, the syringe can connect to the catheter device by a port. The port, and therefore the syringe, is preferably arranged at the proximal end of the catheter device. Even more preferably, the port is arranged between the vacuum pump and the access valve of the catheter device. The syringe is adapted to sample fluids passing within the catheter device. The presences of blood within the catheter device indicates that the thrombus is not sufficiently attached by the suction cup. Inversely, if no blood is detected within the catheter device with the syringe the attachment is sufficient for extraction of the thrombus.

In some embodiments, a control unit may be present.

The control unit may be adapted to receive a signal confirming the anchoring of the medical device to a thrombus. To this end, the medical device may comprise one or several sensors.

The sensor may be adapted for evaluating the change in the vacuum induced by the vacuum pump. When the suction cup is not in sufficient contact with the thrombus, a gap between the surface of the cup and the proximal surface of the thrombus may be present. As a result, blood may be aspirated. The sensor may thus be used to measure the pressure and/or the flow rate in the medical device.

The sensor may be embedded in the medical device, in particular near the distal part. The sensor may be embedded on the proximal area of the flexible line to reduce the weight of the distal part of the medical device. The sensor may be powered by electrical wires. Electrical wires may also be used to transmit a measured signal to a sensor acquisition system.

The pressure may be measured with sensors using hydrostatic gauges, piston technology, and/or mechanical deflection (such as Bourdon tube, diaphragm, and/or bellows), piezoelectric transducers, MicroElectroMechanical systems (MEMs), silicon resonator, variable capacitor transducers, strain gauges, piezoresistive semiconductors, Pirani gauge and/or hot-filament ionization gauge.

The sensor may be adapted to measure a pressure between -1 bar and 0.3 bar, relative to atmosphere, in particular with an accuracy between 0.1 mBar and 100 mBar.

A flow rate can be measured with sensors using magnetic inductive technology, vortex technology, swirl technology, thermal technology, mechatronic spring-based technology, ultrasonic technology, Venturi effect and/or Coriolis effect. The sensor may be adapted to measure flow rates between 0 mL/min and 500 mL/min, in particular with an accuracy between 1 mL/min and 0.001 mL/min, preferably between 0.05 mL/min and 0.005 mL/min.

The sensor may in particular be an electrical sensor. In particular, an electrical sensor may be arranged on or with respect to the suction cup. The penetration of an electric current through a tissue may depend on the tissue type. Hence, by applying and measuring the resulting current when a voltage is applied, it may be possible to determine if the entire surface of the cup is in contact with the thrombus or a vessel wall. To this end, the suction cup may exhibit an emitting probe and a receiving probe.

For example, a current may be between 0.001 A and 1 A in typical tissue when a voltage of 0.03 V and 30 V is applied. The sensor is made of an emitting probe and a measuring probe. The probes may be flat or formed as rods, for example with a height of 0.2 mm and a diameter of 0.3 mm.

Alternatively, electrochemical impedance spectroscopy might be used. In such configuration, a sinusoidal voltage is applied over a wide range of frequency. The measured current response is a characteristic of the tissue.

With the sensors described previously, during the retrieving and the extraction, a change of the values measured may indicate that a part of the thrombus or the entire thrombus is detached from the medical device.

Preferably, a size of the attachment element in when attached to the thrombus is smaller than 3 mm, particularly preferably smaller than 1 mm, even more preferably 0.8 mm, in a direction perpendicular to a longitudinal axis of the distal part of the catheter device.

Preferably, the size of the attachment element is between 0.2 and 5 mm, particularly preferably between 0.3 and 1 mm.

The distal part of the catheter device, in particular the magnetic part with the attachment element, may typically be moved forward mainly by a flow force exerted by blood. When a thrombus is present, the flow may be modified or reduced.

Preferably, the flexible line is attached, with a first portion to a distal portion of the main body. The first portion of the flexible line may be attached inside an internal channel of the catheter main body. A second portion of the flexible line may be attached to the attachment element.

As a result, the distal part of the catheter device may be fixed, via the flexible line, to the catheter main body, in particular in a distal portion. This facilitates operation and manufacturing of the catheter device because the flexible line does-not need to be arranged along the entire length of the catheter device and also does not need to be moved along such a length to retrieve a thrombus.

Preferably, the flexible line is adapted for magneto-fluidic navigation. The distal part of the catheter device may be adapted for being pushed by a drag force exerted by a surrounding fluid, in particular blood.

Preferably, a suction channel is arranged within the flexible line. The flexible line then forms the suction line. This provides for a particularly easy manufacturing of the catheter device because not separate suction line needs to be arranged with respect to the catheter device.

Alternatively, a separate suction line may be used.

In some embodiments, the catheter device may comprise an orientation segment configured to allow revertible orientation of the attachment element from a first position to a second position. At least one of the first position or the second position of the attachment element at least partly deviates from a longitudinal main axis of the catheter device.

The orientation segment is preferably situated more proximally along the catheter device than the attachment element. For instance, the orientation segment can be positioned upstream of the distal part of the catheter device and operated to change the position of attachment element positioned at the distal part of the catheter device. Alternatively, the orientation segment may be coincident with the distal part of the catheter device.

In a general manner, the orientation segment is at least partly flexible or deformable such that it can reversibly bend from the first position (e.g. substantially aligned with the main axis of the main body of the catheter device) to the second position (e.g. at least partly deflected from the main axis). In this instance, the orienting of the attachment element is induced by controlled bending of the orientation segment.

The orientation segment may be made of any suitable biocompatible material which would allow for sufficient bending of the orientation segment. Thus, the orientation segment may comprise a flexible material. Additional or alternatively, the orientation segment may include a shape memory material.

The flexible material may include any one or more of: a polymer, a metal, a resin, a rubber. Preferably the flexible material may include one or more of silicone; polyurethane; polyvinyl chloride; a thermoplastic elastomer; latex; polyethylene; polypropylene; a fluoropolymer.

It is understood that the term "bend" is intended as meaning that the orientation segment at least partly deviates from the main axis of the catheter device.

The orientation segment may be the folding structure as described above in relation to the aspiration catheter. Hence, the orientation segment may for part of the aspiration catheter.

The orientation segment may be, or may further be, the folding structure as described above in relation to the flexible line. Thus, the orientation segment may be part of the flexible line.

The orientation segment may allow for improved orientation of the attachment element. An orientation segment made of polymers, e.g. elastomers such as silicone and/or polyurethane, may be used. Blends and/or copolymers may also be used. For example, the orientation segment may be a polymer insert.

It is conceivable to configure the orientation segment as a portion of the catheter device with reduced wall thickness. For example, the outer tube diameter of the main body of the catheter may be 2 mm with an inner diameter of 1.7 mm. The orientation segment of the catheter device may be a tube with an outer diameter of 2 mm and an inner diameter of 1.8 mm. The orientation segment can have a length between 0.5 mm to 30 mm, preferentially between 1 to 5 mm.

Additionally or alternatively, the orientation segment may have a reduced cross section with respect to the main body. Additionally or alternatively, the orientation segment may have a reduced cross section with respect to the distal portion of the main body.

In some embodiments the orientation segment can be configured as a portion of the catheter with a pleated profile. In this case, the pleats of the orientation segment may change between a contracted and de-contracted configuration to allow bending of the orientation segment, The pleated orientation segment may distribute forces from the activation system more evenly allowing the attachment element to be oriented more smoothly and more precisely.

Alternatively, it is possible to provide an orientation segment having a coiled or spiralled profile. Such a profile is able to deform upon actuation of an orientation system and resume its initial configuration once no longer actuated by the orientation system.

The orientation segment may be made of a material configured to deform or bend upon exposure to a liquid of a predetermined temperature. The material is preferably a shape memory material such as a shape memory Polymer (SMPs) and/or a shape memory alloy (SMA). In this case, the memory shape alloy is configured to adopt a bent configuration (i.e. change from a first position to a second position) upon exposure to a heated fluid. The heated fluid is understood to have a temperature which is at least the transformation temperature of the shape memory material. Preferably the heated fluid may be at a temperature above 37°C and preferably lower than 45°C.

A cold fluid may be applied to revert the orientation segment from the second position to the first position. It is understood that the cold fluid has a temperature which is below the transformation temperature of the shape memory material Preferably the cold fluid may be at a temperature above 32°C and preferably lower than 38°C.

Thus, a fluid may be used to bend the orientation segment from a first position to a second position. The fluid may be hot (i.e. above 37°C); cold (i.e. below 37°); at room temperature.

The catheter device may comprise an orientation system which actuates the bending of the orientation segment. The orientation system may be configured to actively and/or passively induce the bending of the orientation segment between the first and second position.

Additionally or alternatively, the orientation system may move the distal part of the catheter device, preferably the attachment element, more preferably the suction cup, between the first and second position thus inducing the bending of the orientation segment. For instance, the orientation system may comprise a magnetic element configured to be steered and/or guided by an external magnetic actuator.

It is conceivable that the orientation system and/or the orientation segment may be actuated by the actuation system of the catheter device.

The orientation system may comprise an activation component configured to actively bend the orientation segment. Additionally or alternatively, the orientation system may comprise an activation component configured to passively bend the orientation segment. For instance, activation component may include any one or more: a fluid management system; a motor unit; a sensor; a wire; a coil; a spring; a balloon; a magnet; an ultrasound device; a piezoelectric component.

The orientation system may be activated by an ultrasound device. For instance, the orientation segment may comprise a piezoelectric component (e.g. embedded in or attached to the orientation segment). The piezoelectric component may be configured to undergo mechanical deformation upon exposure to ultrasonic waves. The orientation segment being made of flexible material will follow the motion of the piezoelectric component. This consequently induces the displacement of the attachment element.

The activation component may form part of the orientation segment. Alternatively, the activation component may be at least partly embedded in the orientation segment.

In some embodiments, the catheter device may comprise a plurality of orientation segments. The plurality of segments may be at least partly distanced from one another and may be selectively and/or individually bent to position the attachment element; preferably the suction cup.

To avoid any collapse of the orientation segment due to a longitudinal effort induced by the contact with the thrombus or a vessel wall, a reinforcement element might be added in the segment. Preferably, the reinforcement element might be any one or more of a rod, a strip or a ribbon. The reinforcement element may comprise any one or more of a polymer; a ceramic; a metal. The reinforcement element may comprise any combination of a polymer; a ceramic; a metal. For instance, the orientation segment may have a reinforcement element comprising two rods made of stainless steelwith a diameter of 200 um. The rods may be arranged such that they are parallel to the main axis of the catheter body.

Preferably, the suction cup is orientable with respect to the main body of the catheter device and/or a main axis of the catheter device. Preferably, the suction is rigidly attached to the magnetic element. The magnetic element may be adapted to be orientable by an external magnetic field. Orientability may be achieved by a magnetic part which allows orientation (i.e. rotation around an axis or center point as opposed translational movement, generally referred to here as "positioning") in combination with a flexible line which allows for bending with small radius of curvature, preferably a radius of curvature between 1 mm and 5 mm. Additionally or alternatively, the flexible line may be connected to the magnetic part and/or the attachment element via an element which is more flexible than the flexible line. Preferably, the distal part is orientable at an angle 0° to 270° in any direction with respect to the main axis of the catheter device and/or the flexible line.

Preferably, the suction cup comprises a magnetic portion may in particular consist of a magnetic material.

The suction cup may increase the weight of the distal part of the catheter device and thus make magnetic or magneto-fluidic navigation more challenging. The magnetic force induced by the magnetic element when interacting with an external magnetic field may have to overcome the drag force induced by blood and the gravity force induced by the weight of the distal part.

Thus, navigation and/or orientation of the distal part may be supported even when the suction cup and the magnetic part are rigidly connected.

Where the suction cup is orientable with respect to the magnetic part, a magnetic suction cup may also enable orientation of the suction cup independently of the magnetic part. In particular in this configuration, one of the magnetic element and the suction cup may be an electro-magnetic element. In this case, the magnetic attraction of the element which is electro-magnetic may be turned off when no power is supplied. The magnetic element and the suction cup may be attached by an element which allows rotation of the two relative to one another, e.g. similar to a patella.

The magnetic suction cup may comprise or consist of a magnetic material, a combination of two or more magnetic materials, magnetic elements, made of such a magnetic material, embedded into a polymer or /and elastomer matrix, or comprise an electro-magnetic element. The magnetic material may be a permanent hard ferromagnetic material (e.g. Nd-Fe-B alloys and/or Fe-Pt alloys), soft ferromagnetic material (e.g. iron alloys, nickel alloys, and/or cobalt alloys), or ferrimagnetic materials (e.g. iron oxide).

The magnetic part may comprise or consist of an aggregation of magnetic particles, for example superparamagnetic nanoparticles made of iron oxide. The suction cup may be coated to prevent direct contact with a biological fluid and thus avoid corrosion. For example, coating materials suitable to at least partially prevent corrosion include polymers (such as Parylene C), ceramics (such as silica-based ceramics, zirconia-based ceramics, TiO2) and metals (gold, silver).

The suction cup may be connected or connectable by two electrical wires adapted to generate a current within the suction cup. Thus, a magnetic field may be generated in the suction cup, which acts as an electromagnetic element.

The diameter of electrical wires may be between 10 µm and 200 um, preferably between 40 µm and 80 um. The electrical wires may have a flat cross-sectional shape and may be attached to the flexible line or may be formed on an outer wall of the flexible line, e.g. by a conductive coating. A width of the electrical wire may be between 10 um and 200 um. A thickness may be between 1 um and 200 um.

Preferably, the suction cup is orientable by means of a magnetic actuator, which may be part of a system together with a catheter device according to the invention. It is particularly advantageous to orient the suction cup with a magnetic actuator because it may be the same magnetic actuator that may be used for navigation and positioning, thus simplifying the treatment. However, it will be understood that it is possible to use separate magnetic actuators for orientation and/or positioning and/or navigation.

Preferably, the suction cup comprises or consists of a shape memory alloy. As a result, the suction cup may be brought in a second shape, for example at a treatment site and/or upon release from the catheter main body and/or deployment from inside a channel of the magnetic part.

Preferably, the suction cup is expandible, in particular into a deployed shape for attachment to a thrombus. To this end, it is particularly advantageous if the suction cup comprises or consists of a shape memory alloy.

Preferably, the main body comprises a rigid portion. The rigid portion may form a distal-most portion of the catheter main body. The rigid portion can be positioned downstream with respect to an orientation segment configured to allow positioning of the rigid portion. A rigid portion may be used to push the catheter main body, in particular before deployment of the distal part of the catheter device, through a vasculature. Rigid may in particular be understood as more rigid than the flexible line and may include a bending stiffness between 500 mN•mm² and 1400 mN•mm².

Preferably, the suction cup has a shape which comprises a portion which is at least partially conical, parabolic, cylindrical, star shape, flower shape, a shape comprising bellows, and an asymmetric shape. It is also possible that the suction cup as a whole has a shape, in particular when seen along an axis perpendicular or parallel to a main axis, selected from conical, parabolic, cylindrical, star shape, flower shape, a shape comprising bellows, and an asymmetric shape. Any combination thereof is also possible, for example, a star shape when viewed along the main axis and which extends conically or parabolically when viewed along an axis perpendicular to the main axis.

Preferably, the suction cup comprises a sensor. The sensor may be a force sensor and/or an electrical sensor. Preferably, the sensor is formed by an electrical ring forming contacts to perform electrical measurements.

The sensor may be adapted to monitor the attachment of the thrombus to the suction cup. The force sensor may be placed behind the suction cup. When the thrombus is sucked, the thrombus exerts a force the surface of the suction cup which is measurable by the force sensor. The force sensor may use pneumatic load cells, hydraulic load cells, piezoelectric crystal load cells, inductive load cells, capacitive load cells, magnetostrictive load cells, strain gage load cells, force sensing resistors (FSRs), magnetic technology, optical technology and ultrasonic technology.

The force sensor may be adapted to measure a force in the range of 0.1 mN and 0.0001 mN, preferably between 0.05 mN and 0.005 mN. The surface of the sensor may be coated, for example with a polymer or an elastomer such as polyurethane.

The sensor may also be an electrical sensor adapted to measure an electrical current through a tissue when a voltage is applied, which may depend on the type of tissue nature. Hence, by applying a voltage and measuring the resulting current, it is possible to determine e.g. the attachment of the suction cup surface with the thrombus, and /or to differentiate between a thrombus and a vessel wall.

The sensor may also comprise an ultrasound sensor adapted to measure the propagation of ultrasound, e.g. in tissue, around the suction cup in order to identify and/or detect tissue which is in contact with the suction cup. The ultrasound sensor may provide information on a distance to the thrombus, thrombus composition, quality of the contact between the suction cup and the thrombus, and/or thrombus size and/or volume.

Preferably, the suction cup comprises an inner surface with a plurality of suction holes. For example, the suction cup may be formed by a double wall, wherein the inner wall is formed with openings into a space inside the double wall. The space may be an extension of the suction line.

The catheter device may be configured such that the internal channel is adapted to receive the attachment element with the thrombus attached thereto. The thrombus is retrievable, into the channel, by exerting a pulling force on the attachment element via the flexible line.

The catheter device may be configured for remote actuation by an operator. Preferably, the orientation system may be remotely actuated by an operator. This may be achieved by use of a remote control; a wireless controller; a computer; a voice-activation system adapted for allowing input of position parameters and/or actuation commands. Preferably, the catheter device may be operated remotely by magnetic actuation. The remote actuation of the orientation system may induce the displacement of the magnetic actuator.

Additionally or alternatively, an operator may manually activate one or more components of the catheter device. To this end, the system may preferably comprise an interface for a user, for example one or more touch screens, knobs, buttons, levers, switches; sliders; adapted for allowing input of position parameters and/or actuation commands.

The catheter device may be, or may further be, configured for automated operation. Automated operation of the catheter device may be achieved by a sensor and feedback system of the device. For instance, the sensor may be configured to collect data relating to the position of the thrombus. The collected data is communicated to the feedback system which then instructs the actuates the bending of the orientation segment based on the collected the data. It is conceivable that the catheter device may be operated by a controlling unit as described herein below in context with the system of the invention.

The invention is further directed to a system comprising a first catheter device and a second catheter device. The first catheter device may be any catheter device as described herein. The second catheter is configured as a retrieving catheter. The second catheter device is adapted to be delivered along the first catheter device, e.g. wherein the first catheter acts as a guidewire for the second catheter device. The first catheter device is adapted to be retrieved through an inner channel of the second catheter device. The second catheter device is adapted to provide an aspiration for removing a thrombus.

The first catheter device is particularly suited for navigation to a thrombus site and for providing initial attachment to the thrombus. The second catheter device may be more robust and/or provide a stronger aspiration than the first catheter device but may be bulkier. Due to the guiding of the first catheter device, navigation is still possible safely and easily despite the second catheter device's bulkier shape and size.

The invention is further directed to a system comprising a catheter device according to the invention, a controlling unit, and an imaging device. The controlling unit is adapted to navigate the distal part of the catheter device to a target location in a vasculature, in particular a treatment site. A suitable system for navigation is disclosed in WO 2022/157189 which is incorporated here by reference.

The controlling unit may help the magnetic navigation. The concept described helps to find a good equilibrium on the different forces applied on the distal part of the catheter device: flow force, gravity force, controlling force and the magnetic force or other potential forces acting on the distal part of the catheter in order to navigate the distal part of the catheter device along the trajectory path. The system may automatically compute the forces and the relations between the forces and define the forces generated by the system, especially the flexible line force and the magnetic force, to ensure that the resulting force moves the distal part of the catheter device along a predefined trajectory.

This balancing model may allow also to optimize the distribution of forces induced by the magnetic actuator and the controlling line. The balancing of forces may also be beneficial for optimizing system requirements, such as e.g. lower magnetic fields and/or lower controlling line forces.

Preferably, the controlling unit may comprise a processor and/or a memory.

The speed may be at least partially predetermined, automatically determined or manually chosen. It is conceivable to use a combination of predetermined, automatically determined, and manually chosen speeds. For example, the controlling unit may calculate a suitable speed profile based on a planned trajectory in a vessel taking into account data about the flow of blood in said vessel and save the speed profile in a memory. Additionally or alternatively, the speed of the controlling line may be adapted during I an intervention automatically, for example via feedback loop taking into account a planned trajectory and actual position data, and/or manually by a user. To this end, the system may preferably comprise an interface for a user, for example one or more touch screens, knobs, buttons, levers, adapted for allowing input of speed parameters. It is possible to use the same or additional interfaces for inputting further parameters related to control of position and speed of the distal part of the catheter device.

Preferably, the controlling unit is adapted to calculate a magnetic field at a device position in space and/or a force exerted on a magnetic element by said magnetic field when the magnetic element is positioned at the device position in space. The controlling unit may in particular take into account at least one of a position, an orientation, and/or a power of the magnetic actuator. Additionally or alternatively, the controlling unit may be adapted for receiving data from a sensor at or close to the device position, in particular data related to the magnetic field and/or force at the device position.

Additionally or alternatively, the device may calculate at least one of a position, an orientation, and a power of the magnetic actuator suitable to achieve a magnetic field and/or a magnetic force at the device position. The magnetic field and/or magnetic force may be calculated qualitatively (e.g. only a direction) or quantitatively.

In some embodiments, the distal part of the catheter device is adapted to interact with a magnetic field created by an MRI system. Preferably, the magnetic part of the distal part is steerable, guidable and/or orientable by an MRI system.

The invention is further directed to a system comprising a medical device, preferably a catheter device or a distal part of a catheter device according to any one of the preceding aspects, a controlling unit, and an imaging device, wherein the controlling unit is adapted to navigate the medical device to a target location in a vasculature.

A method of retrieving a thrombus from a vessel with a medical device, preferably using a medical device according any one of preceding aspects and in particular a catheter device according to the invention, comprising the steps of
- Introducing the medical device into a vasculature of a patient;
- Navigating the medical device (1) to a target location;
- Optionally, releasing a distal part of the device from a medical device main body,
- Optionally, activating an activation mechanism such that the attachment element is transformed from a deactivated state to an activated state where it is attachable to the thrombus, preferably on a proximal side of the thrombus;
- Attaching the attachment element to the thrombus on a proximal side of the thrombus, preferably by a suction mechanism, preferably including a suction cup arranged on the distal part;
- Optionally, stretching the thrombus to reduce the diameter of the thrombus in a cross-section perpendicular to a longitudinal axis of the vessel;
- Removing the thrombus from the target site by pulling on said thrombus, preferably wherein the thrombus is moved into the medical device main body.

Optionally, the thrombus may be pulled by into a separately configured retrieving catheter within which the medical device is arranged.

In some embodiments the method may comprise or may further comprise a step of flushing the medical device with a solution (e.g. a saline-based solution; heparin-based solution). The flushing of the device may be performed at any point during the intervention. For instance, the device may be continuously flushed with a saline solution to prevent the build-up of blood during the navigation of the device within the vessel. This may help avoid formation of thromboses. In some cases, it may be preferable to perform a flushing prior to anchoring the device to the thrombus. The viscosity of the solution inside of the medical device may impact the time required to reach the distal vacuum. Hence, before the anchoring step, the medical device may be entirely flushed with saline solution or saline solution with heparin preferentially. This may reduce the time required to reach the highest distal vacuum level.

Generally, to reach a distal vacuum, it may be necessary to remove liquids in the catheter device. The removal of liquids may be more time consuming as the viscosity of the liquid increases. For example, to reach a distal vacuum of -0.9 bar it may take up to 180 s if the viscosity is high. Thus, by flushing with a saline solution, the time to reach a similar distal vacuum level may then be reduced to around 30 s. The viscosity coefficient of blood is roughly four times higher than the one of saline solution. Therefore, by flushing, the anchoring quality may be improved.

Preferably, the step of removing the thrombus may be done in a stepwise manner. For example, retrieving steps of 2 mm with a stop of 1 s in between may be performed until the distal part of the thrombus is deblocked, i.e. moves without substantial friction against vessel walls. Alternatively, the retrieving may be done with a forward and backward movement, e.g. of + 2 mm and - 1 mm).

During suction, clot fragmentation may occur it may be necessary prevent such thrombus fragments from clogging the flexible line.

To this end, a sieve may be placed centered inside the suction cup close to its proximal end and, in an aspiration direction, before the flexible line. This sieve may be dimensioned to have a mesh size between 5 µm and 500 um. It is also possible to have a succession of sieves of decreasing size, typically ranging between 5 um to 500 um in order to enable the progressive breakdown of fragments to an acceptable size for the flexible line.

It is also conceivable to adapt a thickness of wires forming such sieves to enable slicing of the fragments. The wires may comprise or consist of nitinol and have a thickness typically ranging from 1 um to 100 um. To prevent blood coagulation on the sieves, the wires may be coated with a hemocompatible coating such as parylene.

One or several sieves may also be used as a constraining spring of a suction cup with the two-way shape memory effect.

Fragments may also be prevented from clogging the flexible line by diverting them away from the entry opening of the flexible line, for example, by a suction cup with a W-shaped proximal end.

A cone, e.g. having a spike shape, arranged with respect to the opening of the flexible line may break down and/or redirect fragments of a thrombus, making it less likely that one or several thrombus fragments gather at the entrance of the flexible line.

Such a spike or cone may comprise a coating to help the fragments glide away, such as parylene. A circular valley, formed a by the cone and an outer surface of the suction cup, may optionally comprise suction channels which may create a depression around the cone and attract the fragments away from the main flexible line entrance.

When present, such channels may have a diameter such as to prevent entrance of larger fragments (which may clog the flexible line). To avoid clogging of these channels, a plurality of channels may be formed between the cone and the suction cup outer surface.

In a further aspect the invention relates to a method for monitoring the attachment of the medical device to a thrombus and monitoring the retrieval of the thrombus. In particular, the method relates to the monitoring of a physical and/or chemical change within the medical device and/or changes to the thrombus. The method preferably using a medical device according any one of the preceding aspects and in particular a catheter device according to the invention, and further uses a controlling unit according to the invention. The method comprises one or more of the following steps of:
- Place the medical device in an occluded artery;
- Determining the position of the thrombus within the vessel of a patient, preferably by marking a position on an image, preferably an image of a segmented vascular network, preferably over the center line, subsequently injecting a contrast solution through the medical device, marking a position corresponding to a distal end of the contrast solution which in turn corresponds to a proximal surface of the thrombus, particularly preferably over the center line, and measuring the distance between these two positions on the image,
- Optionally, move the medical device forward to reduce the distance of the attachment element and the proximal surface of the thrombus based on the determined position of the thrombus, e.g. at a distance of 1 mm to 5 mm, preferably between 2 mm to 3 mm to the position of the thrombus,
- Optionally, check the orientation of the attachment element and/or medical device with or without the magnetic actuator;
- Optionally flushing the device with a fluid;
- Optionally, move the magnetic actuator to the appropriate position for the orientation of the attachment element on the clot;
- Release the medical device/flexible line by a multiple of the theoretical distance between the attachment element and the proximal surface of the thrombus, preferably a multiple between 1 and 5, preferably between 1.5 and 2;
- Optionally, check the position and orientation of the attachment element
- Activate the vacuum in the medical device;
- Check the anchoring quality.

Additionally or alternatively, any of the following steps may be present:
- Measuring the distance separating the catheter device, preferably the attachment element, and the thrombus, preferably the proximal surface of the thrombus;
- Determining the amount of flexible line to be released such that the distance between the attachment element and the thrombus is at least partly reduced; preferably such that the separating distance is closed,
- Positioning the attachment element at the surface of the thrombus for anchoring of the attachment element to the surface of the thrombus;
- Operating the attachment element to anchor the attachment element to the thrombus, e.g. by activating a vacuum and/or monitoring a flow rate;
- Optionally, releasing the thrombus and repositioning the attachment element by use of the magnetic actuator for attaching the attachment element to the thrombus for extraction;
- Actuating the catheter device for retrieving the thrombus from the vessel.

In another aspect, the invention is directed to a pushable catheter device.

Aspiration catheters are known in the art and are used for mechanical thrombectomy. Known catheters have a diameter along their full length which is at least equal or superior to the diameter of a distal-most portion. However, one drawback is that a large catheter is more complex to navigate in tortuous vasculature. The navigation of the aspiration is generally considered complex step of the mechanical thrombectomy procedures. In particular the direct navigation of an aspiration catheter in tortuous cerebral network up to the thrombus may be challenging. Generally, in solutions known in the art, a guidewire is provided up to or through the clot. A micro-catheter is moved on the guidewire, and subsequently the aspiration catheter is moved on the microcatheter. Thus, two additional medical devices are required to for use the aspiration catheter.

The diameter of the microcatheter is larger than the one of the guidewire, and the diameter of the aspiration is larger than the one of the microcatheter. In the devices of the art, the micro-catheter is required as the difference of diameter between the guidewire and the aspiration catheter would otherwise lead to a deviation of the aspiration catheter from the guidewire path.

For example, a microcatheter and aspiration catheter ("Tenzing 7" and "FreeClimb") are disclosed by Settecase *et. al*. (Interventional Neuroradiology, DOI: 10.1177/15910199231177754). The microcatheter has a progressive increase of its diameter.

The object of the invention is to provide a catheter which enables easier navigation.

The catheter device according to the invention, which may optionally be any catheter device as disclosed herein, has a proximal section and a distal section, and a distal head. The proximal section and the distal section define a longitudinal axis and are fixedly attached to each other such as to not allow translation between the distal section and the proximal section.

The distal head is in fluid communication with an internal chan-nel extending through the proximal section and the distal section. The distal section is less rigid than the proximal section. The proximal and the distal section are both pushable.

The distal section may be narrower, i.e. have smaller crosssec-tion, than the proximal section.

In a particularly preferred embodiment, the proximal section has an inner diameter of at least 1.7 mm, and is connected to a distal section with an inner diameter of at least 300 um and an outer diameter of at least 600 µm.

The inner diameter of the distal section may correspond to an outer diameter of a guidewire so as to allow the progression of the catheter device on the guidewire.

The high flexibility of the narrow distal section and its inner diameter more closely matching the diameter of the guidewire improves the navigation of the catheter device and allows easier following of the path defined by the guidewire. In particular, the distal section may allow to pass complex turns.

The catheter device may be manipulated with standard catheter controls such as push, pull and torque movement from the proximal section and/or a handle.

The distal section is more flexible than known aspiration catheters and may create less friction, less resistance than a large aspiration catheter facilitating the navigation due to its reduced size which minimizes surface contact with vessel walls.

The catheter device may be advanced by itself or in conjunction with guidewires and/or microcatheters.

The distal section may have a flexibility/rigidity adapted to minimize the catheter navigation constraints while being rigid enough to transmit the push and torque movements up to the distal-most parts, e.g. a suction cup. Furthermore, the distal section may create vacuum level and/or aspiration flow, and may also provide sufficient tensile strength to pull a clot back.

The narrow distal section is preferably made of polymers such as polyurethanes, polycarbonate-based polyurethanes, polyether-based polyurethanes, polyamides, polyimides, polyolefins, or mixtures thereof. The distal section can be made of a multilayer of polymers.

The distal section may have a reinforcement layer, for example made of a metal such as nitinol. The reinforcement layer may exhibit a braid and/or coil structure. Additionally or alternatively, an internal and/or external coating may be present on the distal section, for example PTFE or hydrogels.

A Magneto-fluidic line may have a flexural rigidity (EI) between 0.002 N·mm² and 0.4 N·mm². A pushable flexible line may have a flexural rigidity between 0.4 N·mm² and 20 N·mm². A rigid line may have a flexural rigidity between 15 N·mm² and 5000 N·mm². For example, a medical device may have a magneto-fluidic line with a flexural rigidity of 0.09 N·mm² and a rigid section with a flexural rigidity of 43 N·mm². As another example, the suction device may have a flexible line with a flexural rigidity of 7.5 N·mm² and a rigid section with a flexural rigidity of 78 N·mm².

A thrombus may have a complex shape and may be blocked in a tortuous artery. The distal section provides sufficient flexibility to more easily position the catheter relative to the proximal surface of the thrombus and thus improve its attachment to the thrombus.

The proximal section may have an outer diameter between 1.2 mm and 2.5 mm.

The distal section may have an outer diameter between 300 µm and 1.5 mm.

The distal section may have an inner diameter between 200 µm and 1.3 mm.

The proximal section may have an inner diameter between 300 µm and 1.5 mm.

The distal head may comprise or consist of a suction cup.

The suction cup may grab the clot and provide a better attachment surface therefor.

The distal head may have an outer diameter between 1 mm and 2.3 mm.

The distal head may have an inner diameter between 0.9 mm and 2.0 mm.

The proximal section and the distal sections may have the same internal diameter, which may be smaller than the internal diameter of the suction cup, thus forming an inner channel with a uniform size.

The proximal section and the distal section may each be rigid enough to be able to be pushed and/or torqued from the device's proximal extremity, while placed in a vasculature, e.g. in the arteries.

The distal head may have an outer diameter between 1 mm and 2.3 mm.

The distal head may have a length between 1 mm and 10 mm.

The distal head may be orientable with respect to the longitudinal axis.

The distal head may comprise or consist of a magnetic element.

In particular, the suction cup may be magnetic, e.g. comprise or consist of a magnetic material. A magnetic actuator may be positioned close to the head of the patient could help to better align the suction cup with the thrombus.

A magnetic suction cup may be made of a magnetic element, a combination of magnetic elements, magnetic elements embedded into a polymer or /and elastomer matrix, and/or an electro-magnetic element.

The magnetic element may be a permanent hard ferromagnetic material, such as Nd-Fe-B alloys and Fe-Pt alloys, and/or or soft ferromagnetic material such as iron alloys, nickel alloys, cobalt alloys, and/or ferrimagnetic material such as iron oxide.

The magnetic part may comprise or consist of an aggregation of magnetic particles, particularly superparamagnetic nanoparticles, e.g. made of iron oxide. The magnetic particles may comprise or consist of a hard or a soft ferromagnetic material.

The magnetic suction cup may comprise or consist of an aggregation of magnetic particles, for example superparamagnetic nanoparticles made of iron oxide.

The magnetic suction cup may be coated to prevent direct contact with a biological fluid and thus avoid corrosion. For example, coating materials against corrosion may be polymers (such as Parylene C), ceramics (such as silica based, zirconium based, TiO2) and/or metals (gold, silver).

The distal section may be flow-driven, i.e. the distal section may be moved forward by the flow. Preferably, for navigation in the blood stream, a guidewire is used.

The catheter device according to the invention may comprise a filter, which may be arranged in the suction cup such as to prevent the aspiration of clot particles into the flexible line/distal section.

The catheter device according to the invention may have a distal section and/or flexible line which is expandable. The increased diameter provided by the expansion may help to prevent occlusion by thrombus fragments and to increase an aspiration flow rate to the proximal section.

In the following, the invention is described in detail with reference to the following figures, showing:
- Fig. 1:: Schematically different attachment principles for devices.
- Fig. 2a-2b:: A schematic representation of a device with a suction mechanism.
- Fig. 3:: A cross-sectional representation of an alternative device with a suction mechanism.
- Fig. 4a-4c:: schematically a device with a catheter and a balloon.
- Fig. 5:: schematically a device with a suction mechanism and an electrical element.
- Fig. 6a-6b:: schematically a catheter device according to the invention.
- Fig. 7a-7g:: schematically shapes of suction cups according to the invention.
- Fig. 8:: schematically a first embodiment a suction mechanism in a suction cup.
- Fig. 9:: schematically a second embodiment a suction mechanism in a suction cup.
- Fig. 10:: schematically a deployment of a suction cup.
- Fig. 11:: schematically the removal of a thrombus from a treatment site.
- Fig. 12:: schematically the retrieval of a thrombus using a retrieval catheter.
- Fig. 13:: schematically an embodiment of a device having a flexible element.
- Fig. 14:: schematically an embodiment of a device having a flexible element and an attachment line.
- Fig. 15:: a detailed view of a suction cup.
- Fig. 16:: another embodiment of a suction cup.
- Figs. 17a-17b:: a further embodiment of a suction cup.
- Figs. 18a-18b:: a further embodiment of a suction cup.
- Figs. 19:: a release mechanism for a suction cup.
- Figs. 20a-20b:: a closure mechanism of a suction cup.
- Figs. 21a-21b:: schematically a deployable suction cup.
- Fig. 22:: schematically retrieving of a thrombus.
- Fig. 23:: schematically a further way of retrieving a thrombus.
- Figs. 24a-24c:: schematically different embodiments of a device with a sensor.
- Fig. 25:: schematically a sensor.
- Fig. 26:: schematically a system including a device according to the invention.
- Fig. 27a-27h:: schematically a series of treatment steps.
- Fig. 28:: schematically a suction cup arranged with respect to a flexible element.
- Fig. 29a-29b:: schematically an auxetic structure.
- Fig. 30a-30b:: schematically a suction cup with a W-shape.
- Fig. 31:: a first embodiment of a catheter device according to further aspect of the invention.
- Fig. 32:: a second embodiment of a catheter device according to further aspect of the invention.
- Fig. 33a-33b:: a third embodiment of a catheter device according to further aspect of the invention.
- Fig. 34:: a fourth embodiment of a catheter device according to further aspect of the invention.
- Fig. 35a-35b:: a firth embodiment of a catheter device according to further aspect of the invention.
- Fig. 36:: a catheter device similar to the embodiment shown in Fig. 6a.
- Fig. 37a-37f: schematically different embodiments of a device with an orientation segment.
- Fig. 38a-38b:: schematically different embodiments of a device with a sensor.
- Fig. 39:: schematically an embodiment of a catheter device with a syringe.
- Fig. 40:: schematically an embodiment of a catheter device with a retrieving catheter.
- Fig. 41:: schematically a catheter device with a reinforcement.
- Fig. 42:: schematically a further embodiment of a catheter device with radiopaque reinforcements and non-radiopaque reinforcements.
- Fig. 43:: schematically an embodiment of a catheter device with a balloon.
- Fig. 44:: schematically a further embodiment of a catheter device with a balloon.
- Fig. 45a-45c:: schematically a series of steps for anchoring and retrieving a thrombus.
- Fig. 46a-46c:: schematically a series of steps for anchoring and retrieving a thrombus.
- Fig. 47:: schematically a retrieval step of a thrombus.
- Fig. 48a-48b:: schematically a device with a distal support.
- Fig. 49a-49f:: schematically a method of retrieving a thrombus.

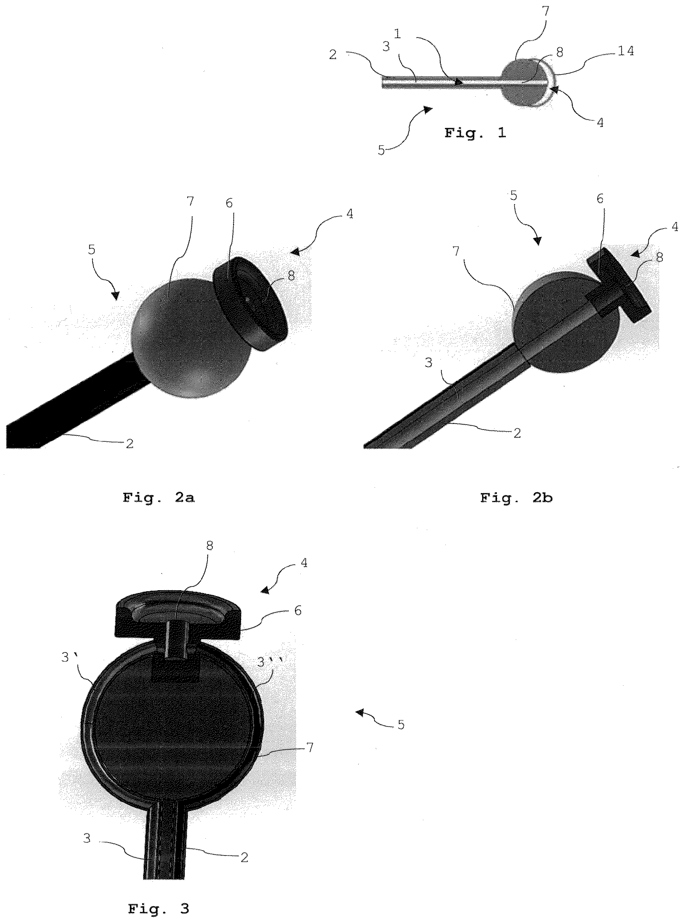

Fig. 1 shows an embodiment of a distal part 5, which may be part of a device (not shown) of the invention. Here, a suction line 3 is arranged within the flexible line 2. The suction line 3 is in fluid communication with an opening 8 arranged at the distal-most end of a magnetic part 7. After removal of a protective layer 14, a vacuum may be applied to the opening 8 via the suction line 3 and may thus be used to provide attachment to tissue, e.g. a thrombus (not shown).

Figs. 2a and 2b show a further embodiment, similar to the embodiment of Fig. 1, of a distal part 5 comprising a suction mechanism. A suction cup 6 is arranged on a distal-most portion of the magnetic element 7 and comprises the opening 8 which is in fluid communication with the suction line 3. The suction cup 6 has a flat inner surface is generally disk shaped. A diameter of the opening 8 is 600 um. The thrombus (not shown) can be pulled onto an inner surface of the suction cup 6 and be held by the suction on the suction cup 6. A suction mechanism as shown here allows to attach and reattach a thrombus repeatedly, for example until secure attachment is provided.

Fig. 2b shows the device of Fig. 2a in a cross-sectional view in a plane parallel to a longitudinal axis of the device. Here, a hollow suction line 3 arranged through the flexible line 2 and the magnetic part 7 is visible. The suction line 3 has an inner diameter of 100 um. The suction line 3 here is configured as a channel within the flexible line 2, i.e. the flexible line 2 serves as a suction channel. The suction channel may, alternatively, be configured as a separate element not incorporated in the flexible line (see Fig. 3).

Fig. 3 shows a distal part 5 for a catheter device (not shown) according to the invention. The distal part 5 comprises a suction cup 6 with substantially the same functionality as the suction cup shown in Fig. 2a. Here, the suction line 3 is arranged on the flexible line 2 and configured as a separate element. The suction line 3 does not penetrate the magnetic head part 7. Instead, a Y-fork divides the suction line 3 into two sublines 3',3'' which are arranged on the circumference of the magnetic head portion 7. Such an arrangement, the magnetic head portion 7 does not need to be modified. The opening 8 is connected to both sublines 3',3", but it will be understood that only one subline may be sufficient for proper functioning. The redundancy thus provides additional safety against malfunctions. The inner diameter of hollow sublines 3',3" is 200 um, and the outer diameter is 300 um. The outer diameter of the flexible line shown here is 700 um. The diameter of the suction orifice 8 is 400 µm.

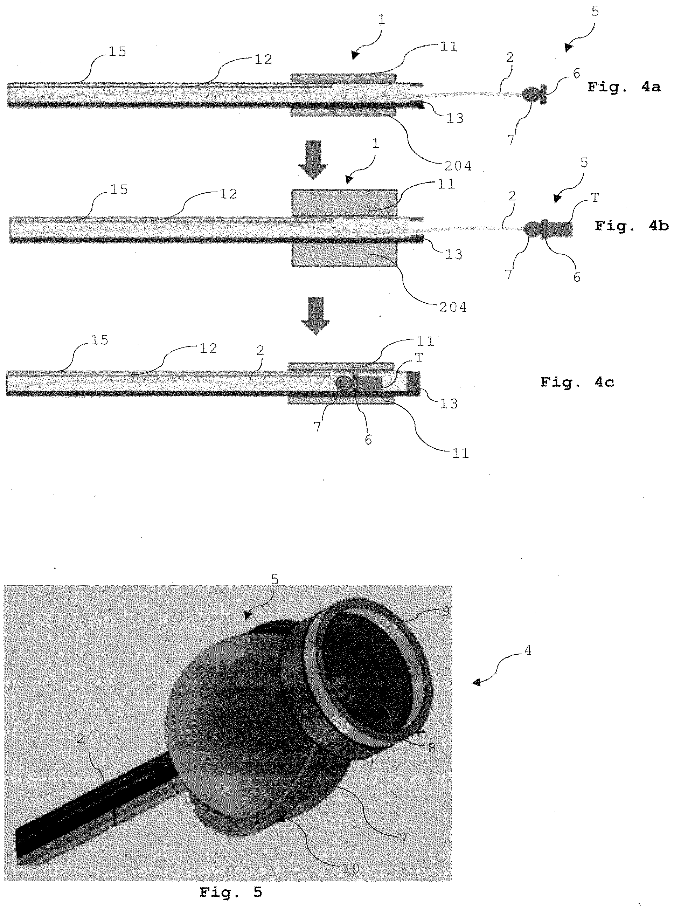

Fig. 4a shows a device 1 with a catheter body 15 according to the invention. The device 1 comprises a magnetic element 6 with an attachment element in the form suction cup 6 forming the distal part 5 of the device. The distal part 5 is attached to the catheter body 15 via a flexible line. It will be understood that the distal part 5 shown here is exemplary and may be replaced with any distal part disclosed herein. The catheter body 15 is more rigid than the flexible line 2 and may thus be used to push the device toward a treatment site. It is conceivable that the catheter body is controlled by a navigation system and guided to a treatment site automatically. The catheter comprises a balloon 11 which can be inflated by means of an inflation line 12. When inflated, the balloon 11 may reduce the blood flow in a vessel in an area where the balloon is located. The reduction of the blood flow may be advantageous both during release of the distal part 5 of the device 1 and during retrieving of the thrombus.

The balloon 11, when inflated, may also provide stability to the catheter body 15 during treatment with the distal part 15. It will be understood that the balloon 11 is advantageous for certain applications but not essential to the invention.

The balloon may be only partially inflated to avoid a total stagnation of the flow. The catheter body 15 further comprises a closure 13 at a distal opening of the catheter body. The closing 13 may be closed when the distal part 15 of the device 1 is housed within the catheter body, either during delivery or after retrieval of a thrombus (see Figs. 4b and 4c).

Fig. 4b shows the device 1 during attachment to a thrombus T. The catheter body is stabilized within a vessel (not shown) by the inflated balloon 11. The closure 13 is opened and the distal part, is released. An external magnet (not shown), for example an MRI system, may be used to navigate the distal part 5 by interaction with the magnetic part 7. Additionally, the magnetic part 7 is orientable by the external magnet such as to orient the suction cup 6. Accordingly, the suction cup 6 may be brought in contact with a thrombus T at a desired angle and position in accordance with the shape of the thrombus.

As shown in Fig. 4c, once the thrombus T is attached to the suction cup 6 of the distal part 5 by means of suction which is provided by the hollow line (not visible) arranged in the flexible line 2, the thrombus may be pulled back by retrieval of the distal part 5 into the catheter body 15. Once the thrombus T is inside the catheter body 15, the closure 13 may be closed again. The balloon 11 is deflated and the catheter device 1 may be retrieved. Enclosure of the thrombus T inside the catheter body provides additional safety because no part of the thrombus T can be accidentally released back into the blood stream.

Fig. 5 shows an embodiment of a device 1 which is based on and substantially similar as the embodiment of Figs. 2a-2b. Here, the device 1 additionally comprises a sensor 9 arranged with respect to suction cup. Here, the sensor is formed as an electric ring connected via wires 10. Once the suction cup 6 is in contact with a thrombus (not shown), a vacuum may be pulled via opening 8 which at least partially pulls the thrombus into the cup and onto the internal surface of the suction cup 6. An electric current may be applied to the electric ring sensor 9 via the electric wires 10 to measure a resistance and confirm that attachment is made to a thrombus (and not another type of tissue). Additionally or alternatively, it may be possible to increase the temperature of the ring by applying a voltage and thus enhance the attachment of the thrombus to the suction cup, for example by partial and/or localized drying. It will be understood that sensor 9 may also be a force sensor to measure a force acting between the suction cup 6 and the thrombus T.

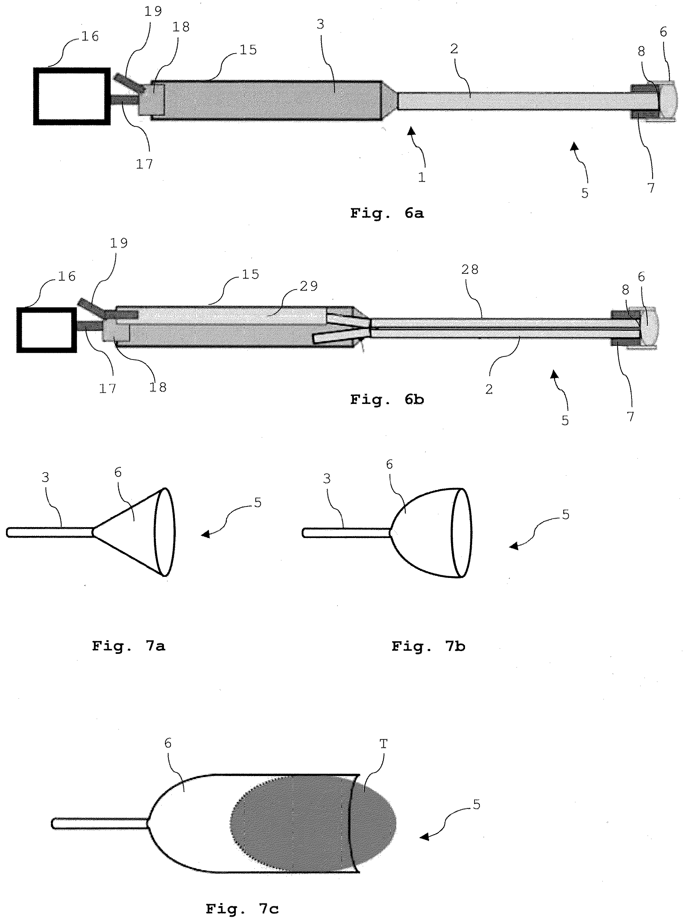

Fig. 6a shows a device 1 according to the invention. The device 1 comprises a rigid catheter body 15 and distal part 5 connected to the catheter body 15 via a flexible line 2. The distal part 5 comprises a magnetic part 7 through which an internal suction line 3 from the flexible line extends and connects an opening 8 inside a suction cup 6. The flexible line 2 is mechanically flexible allowing bending of the flexible line substantially without exerting a force on the magnetic part 7 and the suction cup. As a result, the suction cup 6 is orientable relative to other parts of the device 1, in particular the rigid catheter body 15 and therefore the suction cup 6 is also orientable with respect to a thrombus (not shown) to be treated.

Such an orientable suction cup 6 therefore allows a thrombus with a complex shape to be treated by being brought in contact with a proximal surface of the thrombus more easily. For example, the suction cup 6 is not substantially restricted in its movement by a stiff flexible line.

The device 1 further comprises a vacuum pump 16 providing a vacuum to the opening 8 via flexible line 2. To this end, the catheter device 1 is equipped with an access valve 18 which has connector 17 for the vacuum pump. The access valve 18 fluidly connects the vacuum pump 16/connector 17 assembly with the suction line 3 in the flexible line 2 while allowing insertion of additional instruments such as a guidewire (not shown) or of a fluid via port 19 without entering thereto.

Fig. 6b shows a distal part 5 which is similar to the distal part 5 shown in Fig. 6a. Here, the suction cup 6 is connected to a second flexible line 28 in addition to the first flexible line 2. The first line 2 and the second line 18 may be used to create suction. Therefore, the suction force can be induced in different area of the cup.

The second flexible line 28 may in particular be used as a backup in case of occlusion or breaking of the first flexible line. Therefore, the second flexible line 28 contributes to increase the safety of the assembly of the distal part of the suction distal part 5. The distal part 5 remains attached to the pushing element 15 in case of the breaking between the flexible line 2 and the distal part 5. It is also possible to use the first or second line 2, 28 for the depressurization and the respective other line to inject a solution or to move a tool like a guidewire (not shown), for example. Each line may be connected to a suction cup. As shown here, the device 1 further comprises a vacuum pump 16 providing a vacuum to the opening 8 via flexible line 2, substantially as shown in Fig. 6a also. In addition, the catheter device 1 is equipped with an access valve 18 which has connector 17 for the vacuum pump 16 and a port 19. Here, the port is fluidly connected to the second line 28 via a separate hollow body 29 arranged within body 15.

Figs. 7a-7g shows different embodiments of suction cups. All the shown suction cups provide the functionality of being connectable to a suction line such as to provide suction through an opening to attach to a thrombus. All suction cups shown here are compatible with any device 1 shown herein. The suction cups mainly differ in their shapes, though in individual cases further differences exist as will be apparent from the description. For clarity, identical features comprised in every embodiment described are not indicated and described separately each and every time.

Fig. 7a shows a suction cup 6 having a conical shape. Schematically, a suction line 3 is shown which is connected to the suction cup 6 and opens in an opening (not shown) inside the suction cup 6.

Fig. 7b shows a suction cup 6 having a parabolical shape.

Fig. 7c shows a suction cup 6 having a cylindrical shape. The cylindrical shape allows retaining a thrombus as a whole. As shown in Fig. 7c, the thrombus T is in the process of being pulled in and is still partially arranged outside the suction cup 6. A cylindrical suction cup 6 may also increase the effective suction surface and thus a holding force. The holding force refers to a force between the thrombus T and the suction cup 6. The holding force may be higher than the force required for the retrieving of the thrombus T.

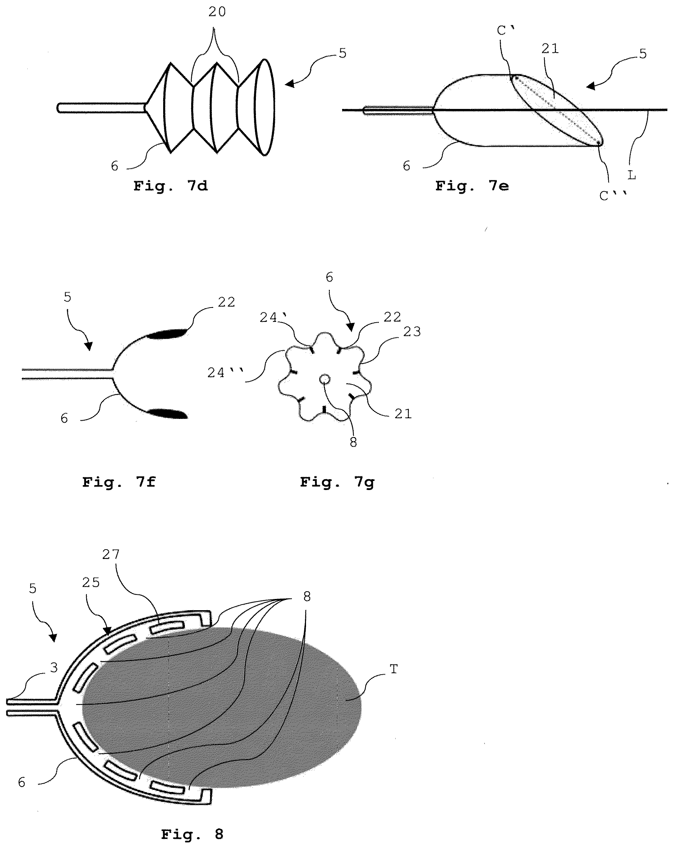

Fig. 7d shows a suction cup 6 having a shape comprising bellows 20. A shape comprising bellows allows for improved anchoring when a thrombus (not shown) has an uneven and/or curved proximal surfaces due to increased surface contact between the suction cup 6 and the thrombus. The shape may thus also provide some attachment without suction and thus act as a "trap", i.e. may help prevent accidental release. The bellows can be placed in various locations of the suction cup 6.

Fig. 7e shows a suction cup having a proximal opening 21 with an asymmetrical shape. The asymmetrical proximal opening 21 is formed, here, elliptically with a first a first vertex C' being arranged at a more proximal position, with respect to a longitudinal axis L of the suction cup 6, compared to a second vertex C". The asymmetrical opening is therefore substantially formed from a conic section of the suction cup in a plane tilted with respect to the longitudinal axis. It will therefore be understood that an asymmetric shape may be obtained with any other suction cup 6 shape and the proximal opening may have a non-elliptical shape depending on the shape of the suction cup 6.

Figs. 7f-7g shows a suction cup 6 having a flower-like shape formed by rims 23 along a longitudinal direction of the suction cup. A flower-like shape allows for compliance to a thrombus with an irregular shape. The rims 23 may additionally pinch the thrombus in a radial and/or tangential direction to provide additional attachment. When viewed in direction of the longitudinal axis (see Fig. 7g), the proximal opening 21 has flower-like shape wherein a rim 23 has a varying distance from opening 8 forming valleys 24' and hills 24". In addition, in the valleys 24' of the rim, ribs 22 are arranged which are collapsible and thus may pinch the thrombus. As a result, when a thrombus is attached by a suction mechanism, it is held by an extra holding force exerted by the ribs 22.

Fig. 8 shows an embodiment of suction cup 6 comprising double wall 25 with a plurality of openings 8. An inner wall 25a of the double wall forms an inner surface of the suction cup 6. The suction line 3 extends into the double wall and is in fluid connection with the plurality of openings 8. As a result, suction attachment is provided at multiple locations on thrombus T which is held more safely. Here, the thrombus T is only partially located inside the suction cup 6 when its proximal face is in contact with the inner wall 25a, i.e. the thrombus T is generally larger than the inside volume of the suction cup 6. It will be understood that such a configuration is compatible with any suction cup disclosed herein and is not limited to the particular shape shown here.

As an exemplary size, the suction cup in the embodiment of Fig. 8 may have diameter of 2 mm and may be adapted to retrieve a thrombus with a diameter of 3 mm and a length of 10 mm.

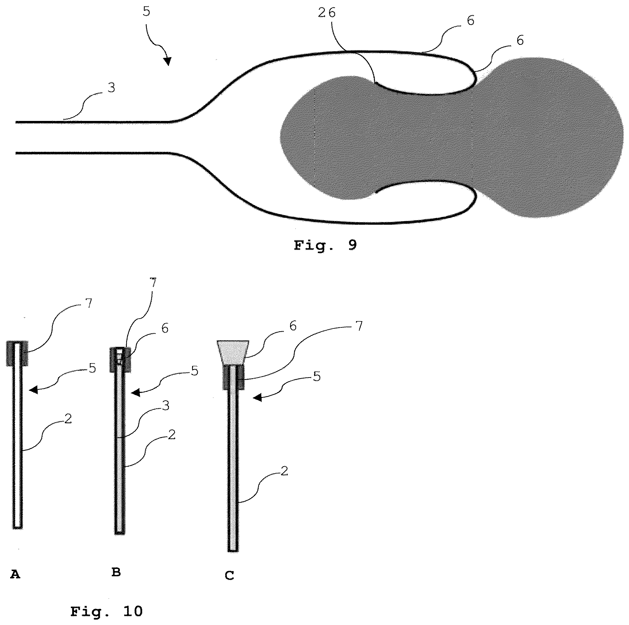

Fig. 9 shows a suction cup 6 having a so-called "fly trap" shape comprising an inner rim 26. The inner rim 26 can provide additional clamping by exerting a radial elastic force and/or by providing a hook mechanism holding the thrombus back, in particular when the thrombus is entered into the suction cup 6 in its entirety.

Fig. 10 shows an embodiment of a distal part 5 for a device (not shown) according to the invention. The flexible line 2 is hollow and is connected to a magnetic part 4, which may be configured according to any embodiment shown herein (see panel A).

A suction cup 6 having a suction line 3 can be inserted via the flexible line 2 (panel B). The suction cup 6 is made of a shape memory alloy and is thus expandible.

As shown in panel C, the suction cup be pushed out of the flexible line 2 through the magnetic part 7 to a position proximal of the magnetic part. There, the suction cup 6 may expand. Pushing of the suction cup may be done with a rod or by injection of a solution, which may also be used to expand the suction 6 in addition or as an alternative to using a shape memory alloy.

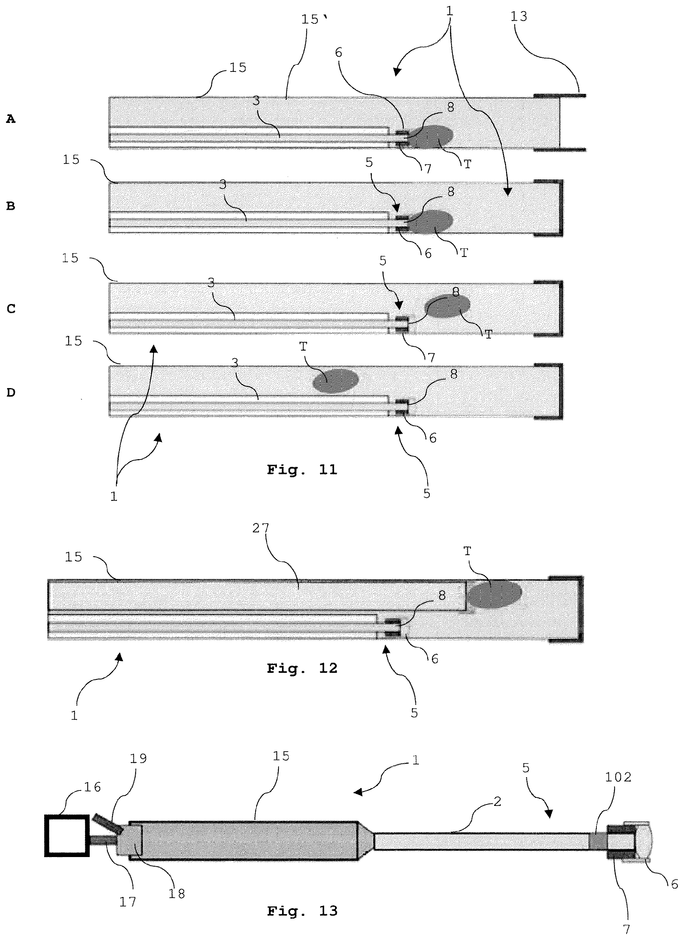

Fig. 11 shows schematically the steps of retrieving a thrombus as it may be performed using a catheter device 1 according to the invention. In a first step, which is not depicted here in Fig. 11 but substantially corresponds to the procedure shown in Figs. 4a-4c, a thrombus T has been attached to an attachment element having a suction cup 6. The suction cup 6 was released from an inside channel 15' of the catheter body 15 and navigated, by means of an external magnet (not shown) interacting with magnetic part 7, to a thrombus T to be removed. Through a suction, thrombus T was attached to the suction cup 6 which was then navigated back into the catheter body 15 by a pulling force exerted on the flexible line 2 and, optionally, by magnetic interaction from the external magnet. As a result, the thrombus T is placed inside the catheter body 15 and attached to the distal part 5 as shown in panel A.

In a second step, shown in panel B, the closure is closed. The thrombus T is now placed inside catheter body 15 and isolated from the blood stream of the patient.

As shown in panel C, the thrombus is then released from the suction cup 6 by turning of the vacuum inside the suction line 3.

As shown in panel D, the thrombus T may then be removed from the suction cup by flushing with saline. Subsequently, the thrombus T is removed by aspiration within the catheter main body 15, such as to repeat a treatment. It would also be conceivable to remove the thrombus T from catheter body 15 by turning off a perfusion and inside the catheter main body 15 such that blood flow may push the thrombus T in a proximal direction. Alternatively, if no other treatment is planned or necessary, the catheter may be retrieved with the thrombus T in it.

It will be understood that the device and method disclosed in Fig. 11 may also be performed using a separately configured retrieval catheter taking the role of the catheter main body 15 described above, and within which a catheter device according to the invention is arranged and moved forward.

Fig. 12 shows substantially the same treatment as shown in Fig. 11, wherein as additionally a retrieving catheter 27 is used which is movable inside the catheter body to connect to the thrombus T and retrieve the thrombus through catheter body. Using a retrieval catheter may be faster and safer than flushing in case further treatment is planned, the distal part 5 may be moved outside the main body 15 again to remove another thrombus (not shown).

Fig. 13 shows a device 1 which is similar to the device 5 as shown in Fig. 6a. Here, the distal part 5 additionally comprises a flexible element 102 arranged between the magnetic part 7 and the suction cup 6. A flexible element 102 may be advantageous when the bending stiffness of the flexible line 2 is not sufficiently low for a particular application. The flexible element 102 may allow for improved orientation of the suction cup 6.

The flexible element may comprise a folding structure which may be easily turned. The folding structure may be made of polymers, e.g. elastomers such as silicone and/or polyurethane. Blends and/or copolymers may also be used. Preferably, the folding structure has at least of 3 folds.

It is also conceivable to configure the folding structure as a portion of the flexible line with reduced thickness. For example, the outer tube diameter of the flexible line may be 600 µm with an inner diameter of 300 um. The flexible element may be a tube with an outer diameter of 600 um and an inner diameter of 400 um.

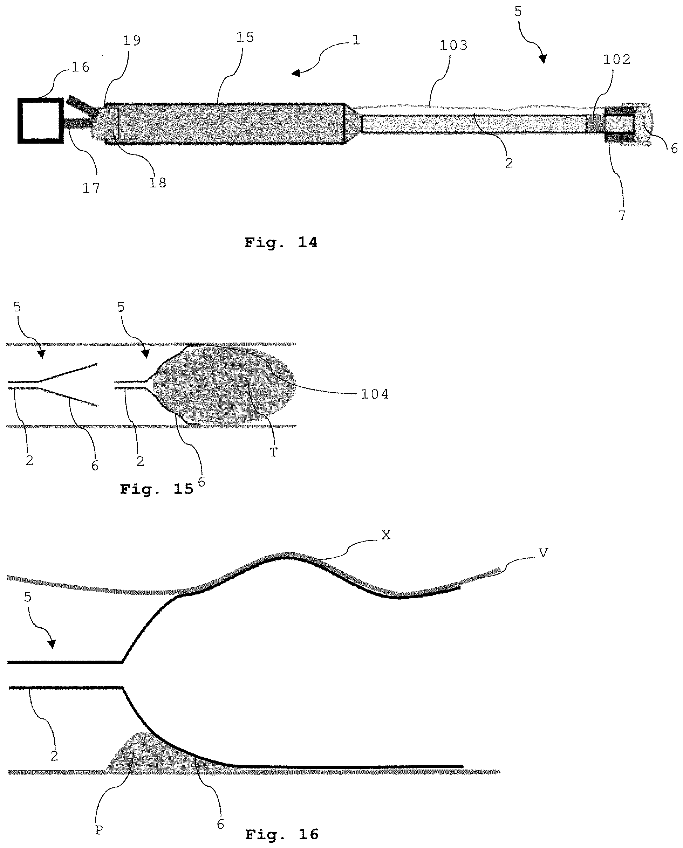

Fig. 14 shows a device 1 with a distal part 5. The device 1 is substantially similar to the device 1 shown in Fig. 13. Here, an attachment line 103 is additionally arranged and connected to the magnetic element 7 and the catheter body 15.

The attachment line 103 may be connected between any part of the catheter body 15 and a part of the distal part 5 of the device 1, e.g. the magnetic element 7 or the suction cup 6.

The attachment line 103 may be advantageous in that it may provide a retrieval option if the flexible line 2 breaks.

In addition, the attachment line 103 may limit the stretching of the flexible line 2. The flexible line 2 may be stretched during retrieving, in particular if attached to a thrombus and especially when unblocking a thrombus. To avoid reaching or exceeding the breaking stress or breaking strain of the flexible line 2, the attachment line 103 may be used. The attachment line 103 may limit the stretching of the flexible line 2.

The attachment line 103 may have a diameter between and 10 µm and 250 µm, preferably between 20 um and 80 um, particularly preferably between 30 um and 50 um.

The attachment line 102 may be made of metal, e.g. a nickel titanium alloy such as Nitinol. If made of a metal, the attachment line 103 may have a diameter between and 10 um and 200 µm, preferably between 20 µm and 50 um.

The attachment line may also comprise or consist.of a polymer, such as an elastomer. For example, polyamides and/or polyester, in particular polyamide 6.6 may be used. When made of a polymer, the attachment line may have a diameter between 10 um and 250 um, preferably between 30 µm and 80 µm. When elastomers are used, said elastomers may have a breaking strain below 100%, preferably between 10 to 70%, such as to avoid that the attachment line is more stretchable than the flexible line.

In some embodiments, more than one attachment lines are used to connect the catheter body 15 and the distal part, preferably exactly two attachment lines 103.

Fig. 15 shows a distal part 5 approaching a thrombus T (left) and attaching to the thrombus T (right). Here, the suction cup 6 has a suction cup lip 104 which extends radially when pressed against an object, here the thrombus T. Thus, the suction cup 6 has larger contact area with the thrombus T.

Fig. 16 shows a distal part 5 with a suction cup 6 and a flexible line 2 inside a vessel V. The vessel V has an irregularity X and a plaque element P. The suction cup 6 is configured to adapt to the irregular surface of the vessel V.

This suction cup may be designed in such a way as to maximize the effective suction diameter in view of the available space inside the artery.

A first embodiment to do so may include using a highly flexible suction cup to minimize an exerted outward force and so as to form a surface matching the inner surface and irregularities of the artery.

A second approach may include a suction cup which.is adapted to exert a sufficient outward force to restore the original lumen of the vessel. This approach, which maximizes the suction diameter, is similar to the lumen restoration achieved with a stent. To this end, the suction cup may have an initially dome-shaped then cylindrical lip towards the end and made of non-porous polyurethane.

To progressively adjust its flexibility, the thickness and/or hardness of the suction cup may be reduced progressively towards the distal end. The hardness may range from shore A0 and D100 and the thickness may be in a range from 50 um to 500 um. A supplementary expansion force may be provided by embedding a superelastic mesh made of nitinol wires ranging from 25 um to 200 um diameter and arranged in a tuned structure to reach the desired expansion force and radial distensibility. This may be, for example, a honeycomb or diamond-shaped structure. To achieve the super-elasticity inside the human body, the selected nitinol wire needs to be tuned to have austenite finish transition temperature below 37°C, preferably around 22°C.

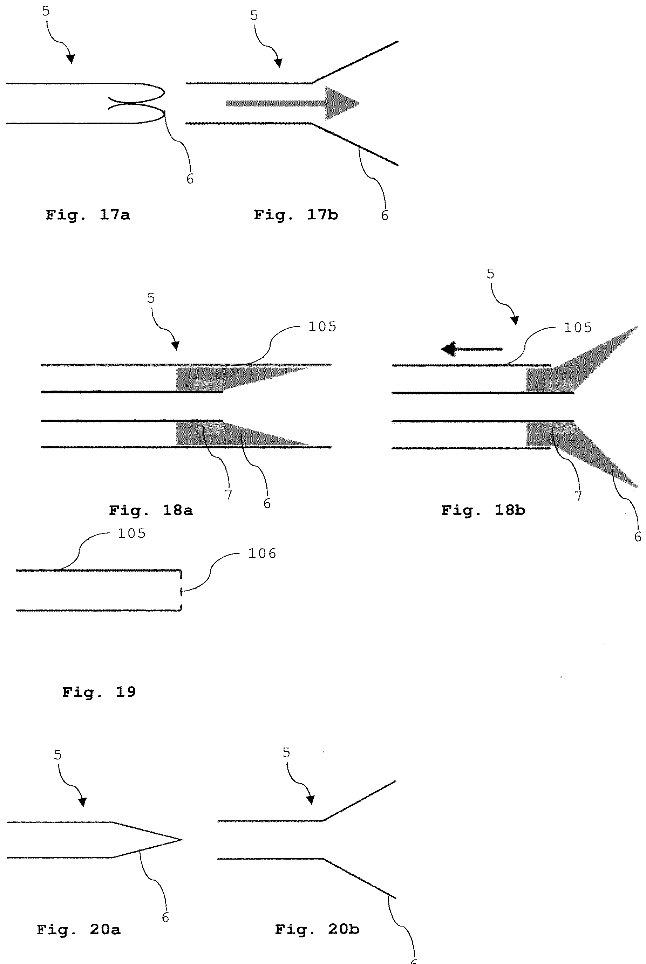

Fig. 17a shows a suction cup 6 which may be deployed using a stable and an unstable mechanical equilibrium position. Here, the suction cup 6 is folded toward the flexible line and is held, in a spring-like manner, and resting on itself in an unstable equilibrium. The suction cup 6 is prevented from deploying.

As shown in Fig. 17b, when deployed, the suction cup 6 may move back to its stable deployed state due. The addition of an extra force, such as hydraulic flow, disrupts the unstable equilibrium (as shown in Fig. 17a) and forces the suction cup to open.

Fig. 18a shows a distal part 5 with a suction cup 6 arranged inside a sheath 105. Here, the magnetic element 7 is embedded in the suction cup 6.

Fig. 18b shows the retraction of sheath 105 which causes the suction cup to expand.

Fig. 19 shows a sheath 105 having a breakaway cap 106 which is adapted to be broken and opened. A suction cup (not shown), in particular a suction cup as shown in Fig. 17a, may be arranged within the sheath and may be deployed automatically when the breakaway cap 106 is opened.

Fig. 20a shows a deployable suction cup 6 which is held shut by a depression inside the lumen of the distal part 5. As shown in Fig. 20b, if the depression is released or a pressure is applied, the suction cup 6 is deployed.

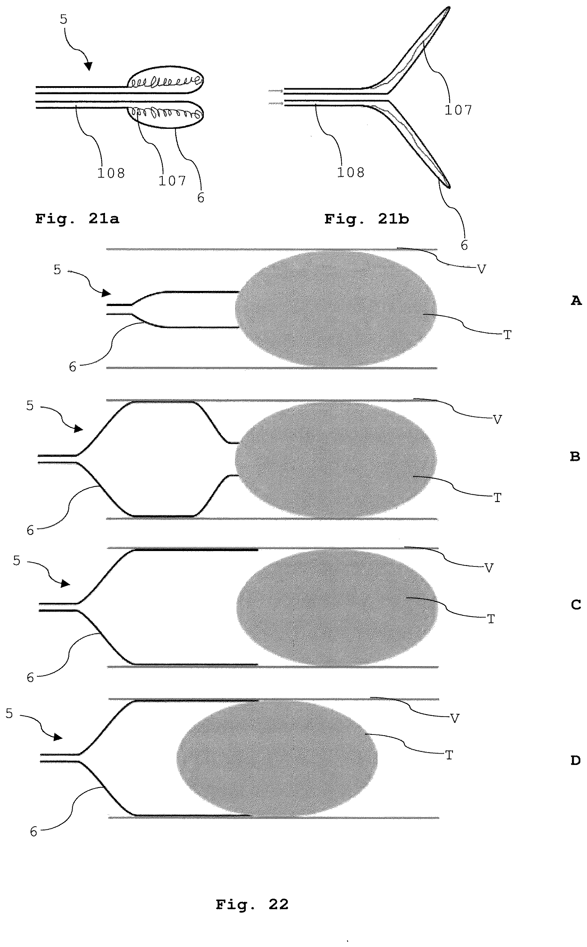

Fig. 21a shows another embodiment of a deployable suction cup 6. Here, the suction cup 6 is configured as an inflatable suction cup 6 which may be inflated by injection of a fluid through an inflation tube 108, here arranged in the side wall of the distal part 5. Furthermore, a spring 107 is arranged inside the suction cup 6 to further assist in deployment.

Fig. 21b shows the suction cup of Fig. 21a in its deployed state.

Fig. 22 shows schematically the deployment of a suction cup 6 which has auxetic properties. In an initial, undeployed state as shown in panel A, the suction cup 6 is arranged on a thrombus T in a vessel V and initially attached via suction action.

As a result, and as shown in panel B, the suction cup 6 expands and comes in contact with the wall of the vessel V. The friction against the vessel wall may be sufficient to further expand the suction cup 6.

Therefore, as shown in panel C, the suction action may be stopped and the suction cup 6 may be released from the thrombus T. Nevertheless, due to the friction against the vessel wall, the suction cup 6 fully expands.

As shown in the panel D, the expanded suction cup 6 has larger opening, compared to the initial state (see panel A) and thus provides a larger surface for attachment to the thrombus T.

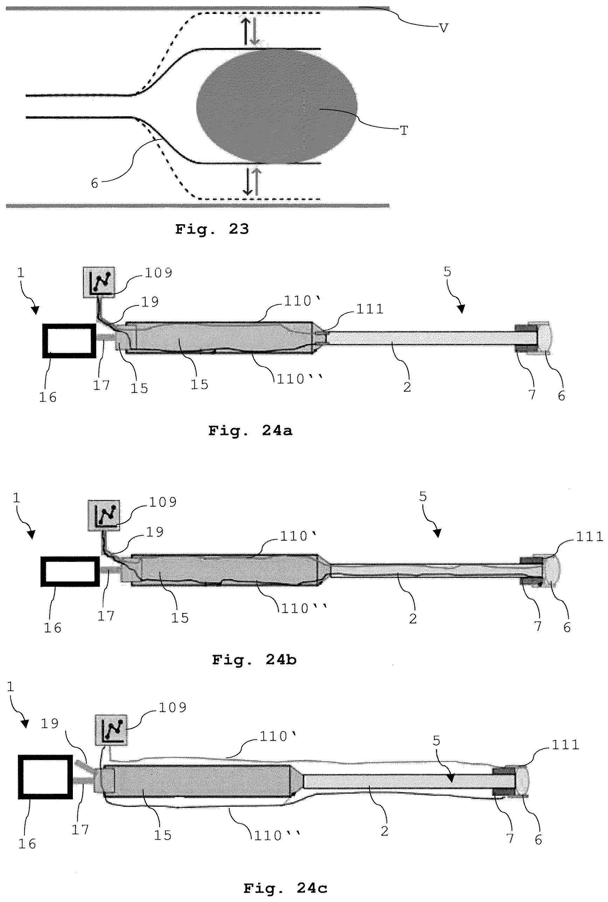

Fig. 23 shows a suction cup 6 which comprises a shape memory allow with a two-way shape memory effect. The suction cup 6 may be deployed from an initial position for navigation to a deployed position using a shape memory effect. Once a thrombus T is inside the suction cup 6, a reverse shape memory effect may be employed to close the suction cup 6 and hold the thrombus.

Fig. 24a shows a further embodiment of a device 1 having a main body 15 and a distal part 5 with a flexible line 2. The device 1 is substantially similar as the device shown in Fig. 6a, and identical features will not be described again for clarity. Here, the device further comprises a sensor acquisition system 109 which is connected to a sensor 111 via electrical wires 110, 110". The electrical wires 110, 110" are arranged within the main body 15 and inside the flexible line 2. Here, the sensor 111 is a force sensor measuring a pulling force on the distal part 5, allowing to determine the attachment of the suction cup 6 to a thrombus (not shown). Additionally or alternatively, a flow sensor, a temperature sensor, or a pressure sensor may also be used. The sensor 111 is arranged on a distal end of the main body 15. The sensor acquisition system 109 may be arranged on the main body 15 or elsewhere with respect to the device 1.

In particular, force sensors as described in WO 2022/268956, which is incorporated here by reference, are suitable for the embodiments shown herein.

Fig. 24b shows a device 1 which is substantially similar to the device shown in Fig. 24a. Here, the sensor 111 is arranged on the magnetic element 7 and is configured as a pressure sensor to detect the pressure in the suction cup 6 such as to determine proper attachment to a thrombus (not shown). Alternatively, a force sensor may be used to monitor the attachment of the suction cup 6.

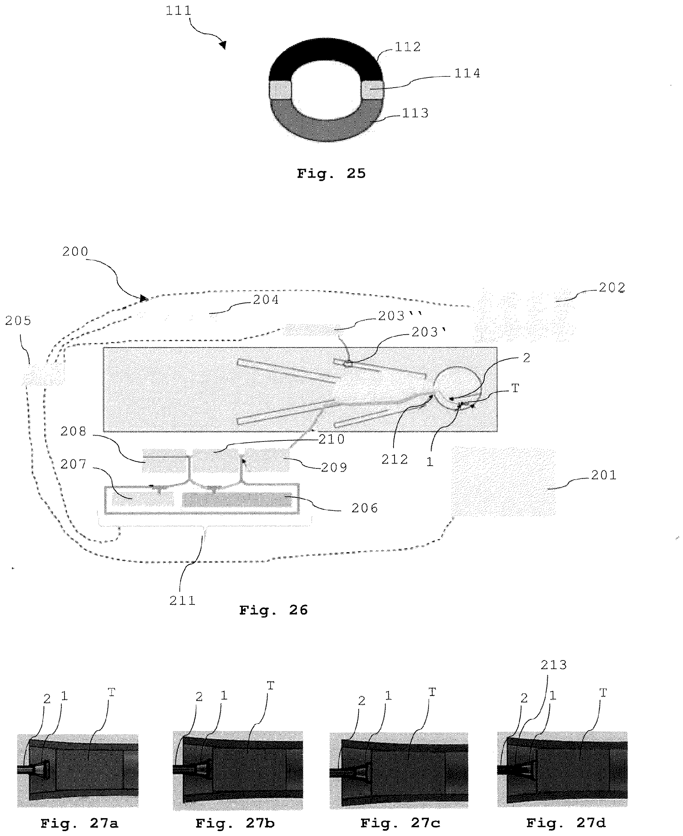

Fig. 24c shows a device 1 similar to the devices shown in Figs. 24a and 24b. Here, the wires 110', 110" are arranged outside the main body 15 and flexible line 2. The sensor 111 is formed by electrical contacts on the suction cup 6 which form emitting and receiving probes (see Fig. 25). A voltage of 0.1 V may be applied and the resulting current is measured and may be, for example, 0.02 A. The penetration of the current in the tissue is dependent on the type of tissue and therefore, the sensor acquisition system may determine what type of tissue is in contact with the suction cup.

Fig. 25 shows an electrical sensor 111 as may be used in the embodiment shown in Fig. 24c. A first probe 112 and a second probe 113 are separated by an insulator 114. The insulator may be formed by the suction cup 6, if the suction cup 6 is insulating. A voltage may be applied between the first probe 112 and the second probe 113 so as to measure the resistance and/or impedance of a tissue attached thereto.

Fig. 26 shows, in an exemplary fashion, a system 200 used to treat a patient and which operates a medical device 1. The medical device may be any device disclosed herein.

The magnetic guidance system 200 shows here comprises a magnetic actuator 202, a controlling unit 205, and an activation system 211 for the medical device 1. The system here further comprises a tracking system 204, an imaging system 201 and a patient sensor 203', 203'' though it will be understood that these latter features are not required to operate the medical device 1.

The controlling unit 205 allows for planning of the navigation, the control of the different elements and the processing of data sent by each element. The navigation planning may be displayed to an operator on a display interface such as a screen; a headset (e.g. virtual glasses).

The controlling unit 205 may further allow balancing the forces exerted on the medical device 1 such as to follow a certain pre-defined a trajectory.

The imaging system 201 may be used to generate the 2D and 3D images of the patient anatomy including the vascular network. The imaging system may be used to track the medical device 1, in particular its distal part and/or a retrieving catheter position. The imaging system 201 may be used to review the blood perfusion of the vascular network.

The tracking system 204 allows to monitor the positions of the different elements of the system. For example, the tracking system may allow to determine the position of the different elements relative to the patient. The tracking system may also allow to follow movements of the patient.

The tracking system may further be used to localize and register the positions of the different element of the system and of the patient, such as patient anatomy, magnetic element, patient table, imaging system, and/or patient sensor.

The magnetic actuator 202 may create a magnetic field which,may be used to operate the medical device 1 and in particular its distal part. The magnetic field is used to orientate the distal part of the medical device and / or to move the distal part. The magnetic actuator 202 may comprise or consist of any magnet known in the art, in particular of a permanent magnet, an electro-magnetic coil and/or and electro-permanent coil. The magnetic actuator may be arranged on a robotic arm (not shown).

The magnetic field may exert a force on the distal part of the medical device 1. The force may be used to attract or repulse the distal part of the medical device 1.

The patient sensor 203', 203" may be used to monitor physiological parameters of the patient. For example, a blood pressure and/or the cardiac rhythm may be monitored. In particular, the systole-diastole cycle may be monitored to synchronize the displacement of the medical device 1.

The activation system 211 may allow controlling the displacement of the medical device 1, in particular of the distal part, and the activation of different functions such as suction, injection of a solution, and/or the displacement of the retrieving catheter. The activation system here comprises three actuators 208, 209, 210 which are connected to a pump 206 for injection of a solution and a vacuum pump 207.

The actuators 208, 209, 210 of the activation system 211 may enable the displacement of the medical device 1, in particular forward and backward along a vessel direction. The displacement may have a predetermined velocity, for example between 0.1 cm/s and 30 cm/s, preferably between 2 cm/s and 10 cm/s. The actuators 208, 209, 210 may also allow to stop and restart the navigation of the medical device 1. To move the medical device 1 forward and backward, any one of wheels, trails, and/or clamps may be used.

A part of any of the actuators 208, 209, 210 and/or a part of the medical device 1 may be filled with a solution, for example a saline solution or a heparinized saline solution.

It will be understood that the system 200 does not necessarily comprise three actuators 208, 209, 210 as shown here, but may comprise only one actuator or several actuators, in particular to control the displacement of different elements of the system 200, e.g. aspiration of the medical device 1, or other elements of the device 1 such as or the retrieving catheter (not shown) and a rod (not shown) used to rigidify the medical device.

The actuators 208, 209, 210 may be used to move the medical device backwards when anchored to a thrombus. The actuators 208, 209, 210 may be configured to move the medical device 1 at a velocity between 0.1 mm/s and 10 mm/s. To unblock the thrombus, the actuators 208, 209, 210 may induce a back-and-forth movement of the medical device, preferably with a short amplitude of movement, e.g. between 0.5 mm and 3 mm. The back-and-forth movement may be repeated at least three times, preferably between five and ten times. The back-and-forth movement may be coupled with a variation in of the vacuum level.

The vacuum pump 207 may be used to induce a depressurization in the medical device 1, e.g. to activate a suction cup. Depression may also be induced, additionally or alternatively, in a retrieving catheter. One or several additional pumps (not shown here) may be used. Preferably, a total of two pumps are used. The pump may be adapted to create a vacuum between -1 bar and - 0.5 bar, preferably between -0.98 bar and -0.85 bar, relative to atmosphere. The vacuum level may be increased in a pre-defined manner and/or with a certain frequency.

The pump for injection of a solution 206 may allow to inject a solution into the lumen of the medical device 1 or the retrieving catheter. The solution injected may be a saline solution, a heparinized solution, and/or a radiopaque solution. The pump may inject the solution with a flowrate between 1 mL/min and 300 mL/min, preferably between 20 mL/min and 100 mL/min. The pump may inject the solution continuously or in a certain frequency. In case of obstruction of the suction cup or the flexible line, a saline solution may be injected. Additionally or alternatively, a rod-like structure (e.g. guidewire) may be moved up to the distal part.

To connect each element of the activation system 211, valves may be used. Valves with multiple channels may be used to switch from one function to another function. Hemostatic valves may be used to avoid leaks. The different valves may be controlled manually or automatically. In particular, the valves may be operated and/or switched by the controlling unit 205. The inner diameter of a valve in this context may be between 0.2 mm and 5 mm, preferably between 1 mm and 3 mm.

The different elements of the activation system 211 may be controlled manually or automatically. The controlling unit 205 may control the different elements according to instructions by an operator.

The activation system 211 may receive information sent by the different elements such a sensor which is in operable connection with the medical device, and may also process such data.

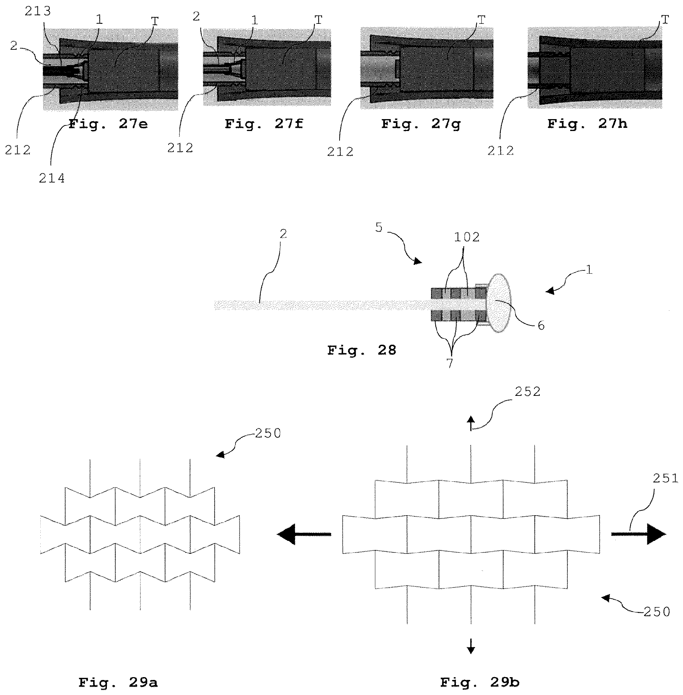

Furthermore, the system 200 is adapted to position an aspiration catheter 212 automatically. Here, the medical device 1 may be placed and attached to thrombus T so to form a guiding point for the aspiration catheter. Optionally, a guidewire (not shown here) may be inserted in the flexible line 2 to increase the flexible line's stiffness. The medical device 1 may be attached to the thrombus by suction as described herein. As a result, the flexible line 2 forms a guiding line for the aspiration catheter 212 which may be advanced the thrombus site for removal of the thrombus (see Figs. 27a-27h).

Preferably, the distal part of the catheter device wherein the magnetic part is adapted to interact with a, preferably external, magnetic field.

Preferably, the activation mechanism comprises a mechanism adapted to release at least the attachment element from a storage area of the catheter device, preferably of the catheter main body.

Preferably, the catheter device further comprises a sensor, preferably arranged in the distal part of the catheter device.

Preferably, a size, preferably a maximum size of the attachment element, in a direction perpendicular to a longitudinal axis of the catheter device or the distal part of the catheter device is smaller than 3 mm, preferably smaller than 1 mm, particularly preferably smaller than 0.8 mm, when attached to the thrombus.

Preferably, the attachment element is smaller, in a direction perpendicular to a longitudinal axis of the catheter device or the distal part of the catheter device, than a maximum size of the catheter device or the distal part of the catheter device when attached to the thrombus (2).

Figs. 27a-27h show schematically a treatment of a thrombus site, by removal of thrombus T, with a medical device 1 according to the invention and using an aspiration catheter 212. It will be understood that any medical device 1 having a flexible line 2 disclosed herein is suitable to be used for this treatment. As such, features of the medical device 1 are not repeated here for clarity.

Fig. 27a shows the medical device 1 being navigated to a site with thrombus T. The thrombus T is blocking the vessel and intended to be removed.

As shown in Fig. 27b, the medical device 1 is positioned on a proximal surface of the thrombus T and then anchored, as shown in Fig. 27c.

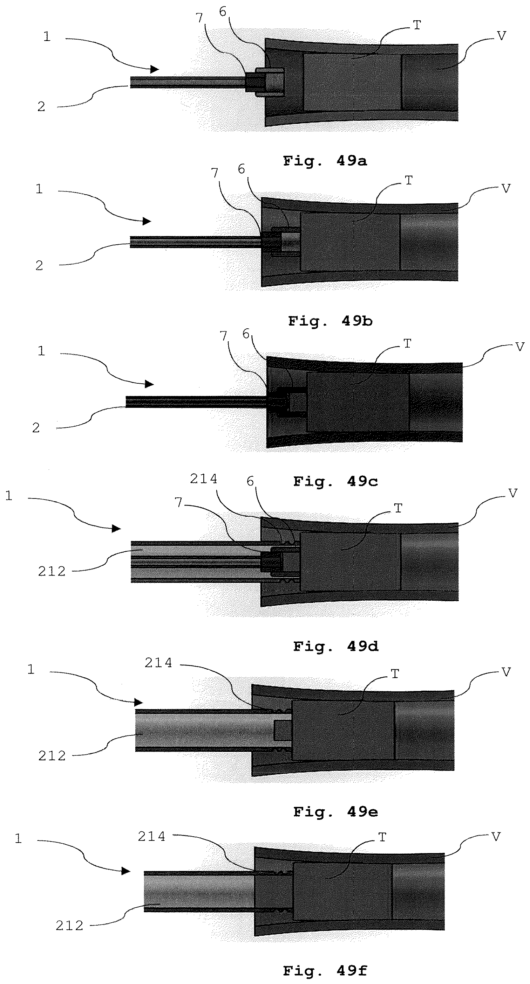

Subsequently, as shown in Fig. 27d, a guidewire 213 is inserted through the flexible line 2 so as to rigidify the flexible line 2. It will be understood that the use of a guidewire is optional and, while advantageous in some scenarios, may not be required (see Figs. 49a-49f), in particular depending on the design of the flexible line, e.g. if a reinforced flexible line is present (e.g. reinforced with a coil).

An aspiration catheter 212 may then be advanced to the thrombus T along the flexible line 2. The guidewire 213 may provide additional rigidity which may assist the guiding of the aspiration catheter 212. The aspiration catheter has a distal portion 214 which has a higher flexibility than a catheter main body, here for example due to ridges on the catheter surface. The flexible distal portion 214 enables, when necessary, the orientation of the aspiration catheter 212 to attach to a thrombus surface.

As shown in Fig. 27f, the guidewire may be removed before, as shown in Fig. 27g, the medical device 1 is also removed through the aspiration catheter 212.

Finally, as shown in Fig. 27h, the thrombus T may be removed by aspiration into the aspiration catheter 212.

Fig. 28 shows a medical device 1 wherein a flexible element 102 (similar to the embodiment shown in Fig. 13) comprises two fractions which are alternatingly arranged with fractions of the magnetic part 7. There may be more than two fraction each. In other words, different magnetic elements 7 are separated by flexible elements 102.

This design may allow the bending of the distal portion 5 in curved vessels. The flexible element 102 here consists of polypropylene. However, the flexible element may comprise or consist of other polymers, for example elastomers such as polyurethane, silicone, or a mixture or blend of polymers.

A length of a fraction of the flexible head 102 are 1 mm for a proximal fraction and 2 mm for a distal fraction, respectively, but may be between 0.5 mm and 4 mm, preferably between 1 mm and 3 mm. The fractions may also have identical lengths. The magnetic element 7 may be made of metal or a mixture of a metal and a polymer. The length of each fraction of the magnetic element 7 may be between 50 µm and 800 µm, preferably between 100 um and 400 um. Here, each fraction has a length of 200 um. The fractions of the flexible element 102 and of the magnetic part 7 are glued together, though other assembly methods such as welding are also conceivable.

Fig. 29a shows a re-entrant honeycomb structure 250 having auxetic properties in its relaxed state.

Fig. 29b shows the structure of Fig. 29b under tensile load 251. As a result of the tensile load 251, and expansion 252 in a direction perpendicular to the tensile load occurs.

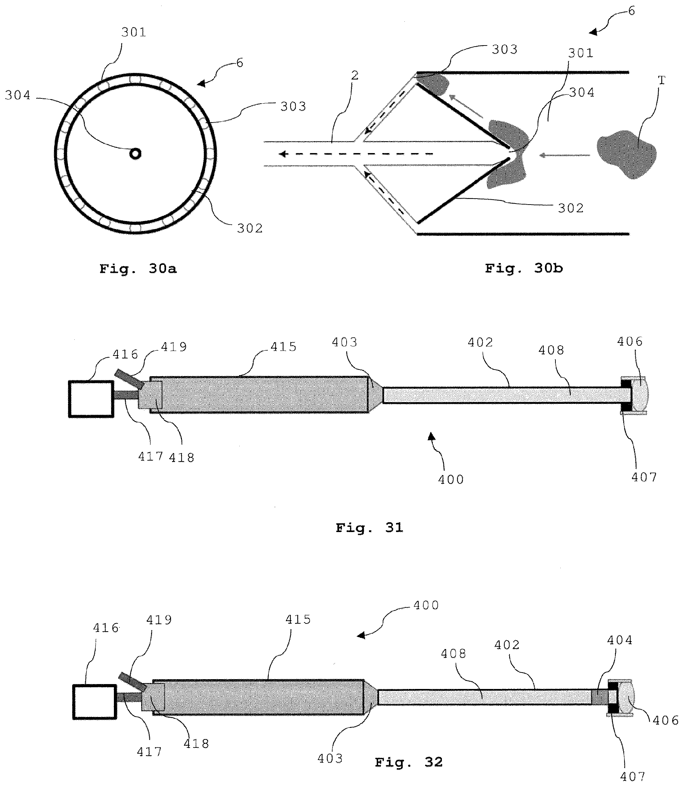

Fig. 30a and 30b show another embodiment of a suction cup 6. The suction cup 6 is similar to other embodiments shown herein, e.g. in Figs. 7a-7g.

In a view along a longitudinal axis and in a direction toward an opening of the suction cup 6, as shown in Fig. 30a, an outer cup surface 301 having a generally cylindrical shape is visible as projection. Here, the suction cup 6 further has an inner cone 302. Between the inner cone 302 and the outer cup surface 301, channel openings 303 are arranged. A main opening 304 is arranged at a central location of the inner cone 302 and leads into the flexible line (see Fig. 30b).

Fig. 30b shows the suction cup 6 of Fig. 30a in sectional side view. The inner cone 302 with central opening is arranged such as to point towards an outside direction of the suction cup 6 and opens conically toward an aspiration direction. Channel openings 303 are in fluid communication with the flexible line 2 and are thus subject to the same aspiration action as central opening 304. The inner cone 302 is adapted to break a larger thrombus T into smaller pieces which are redirected towards channel openings 303. As a result, clogging of the suction cup 6 may be avoided. The inner cone 302 and the outer cup surface 301 form a W-shape. The inner cone 302 is coated with parylene, a hemocompatible coating, to reduce friction with fragments of a thrombus T.

Fig. 31 shows a catheter device 400 having a rigid proximal section 415 and a flexible distal section 402 connected via a connector 403. A suction cup 406 is arranged at a distal end of the distal section 402. A channel 408 spans through the proximal section 415 and the distal section 402. The proximal section 415 has an internal diameter, which forms the inner channel 408, of 0.5 mm and an outer diameter of 2 mm. The distal section has an inner diameter of 0.5 mm, which forms an extension of the inner channel 408 which has a uniform size throughout the proximal section 415 and the distal section 402. The outer diameter of the distal section is 1 mm. The suction cup 406 is adapted to come in contact with a thrombus (not shown). An outer diameter of the suction cup 406 is 2.2 mm and an inner diameter of the suction cup 406 1.5 mm. The length of the suction cup section is 2 mm. The catheter device 400 is equipped with an access valve 418 which has connector 417 for a vacuum pump 416. The access valve 418 fluidly connects the vacuum pump 416/connector 417 assembly with the inner channel 408 in the proximal section 415 and distal section 402 while allowing insertion of additional instruments such as a guidewire (not shown) or of a fluid via port 19 without entering thereto. A connector 407 is arranged proximally of the suction cup 406 to facilitate navigation and orientation of the suction cup 406.

It will be understood that the size of the medical device may be adapted to address other indications and/or other anatomies than the brain.

Fig. 32 shows a second embodiment of a catheter device 400 which is similar to the embodiment shown in Fig. 31. Here, a flexible element 404 is arranged proximal of suction cup 406 and connector 407 to facilitate its orientation. The flexible element may be made a folding structure which can easily turn. The folding structure may be made of polymers or elastomers, for example silicone, polyurethane, or a combination of polymers. The folding structure may be made at least of three folds.

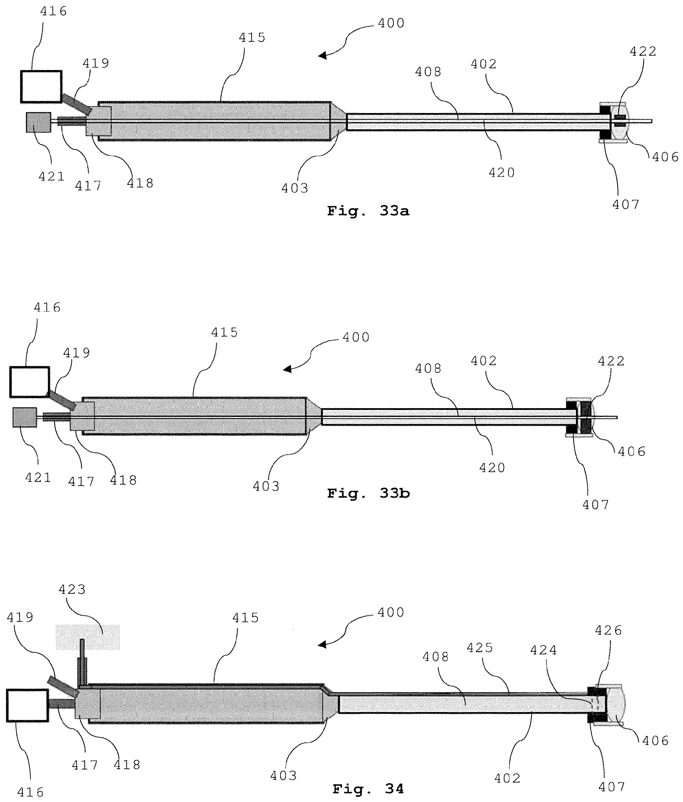

Figs. 33-35 show different embodiments of a catheter device which are similar to the embodiments of Fig. 31-32. For clarity, identical features with identical reference numerals are not described repeatedly.

Fig. 33a shows a catheter device 400 being used in conjunction with a guidewire 420 which is arranged within channel 408. The guidewire 420 has an activatable distal part 422 and a guidewire actuator 421.

As shown in Fig. 33b, the activatable part 422 may be expanded using the guidewire actuator 421.

In an occluded artery, the suction cup 406 may be oriented in a downward direction, due to gravitational forces acting on its weight. For optimal anchoring to the proximal surface of the thrombus (not shown), the cup is advantageously aligned with thrombus. Guidewire 420 may provide an alignment help. The activable structure 422, when activated, increases the distal volume of the guidewire which increase the contact surface with an inner part of the suction cup 406, thus allowing positioning and orientation by means of the guidewire 420.

The activable structure 422 might be an opened structure made, for example, from a nitinol frame, for example activated by Joule effect or a balloon which may be filled with a saline solution, for example.

The guidewire 420 may have an inner lumen with a diameter of 200 um. The guidewire 420 may have an outer diameter between 300 µm and 500 um. The guidewire 420 may have a different outer diameter in a distal part than in a proximal part, for example 500 um in the proximal part 300 um in the distal part. A balloon made of polyurethane with a thickness of 100 um may be used.

Fig. 33b shows the catheter device 400 of Fig .33b in an activated state, i.e. where the activatable element 422 is expanded.

Fig. 34 shows a catheter device 400 further having an orientation system 424 connected to remote actuation controller 423 via a cable 425. The orientation system 424 may be arranged around or inside the suction cup 406 to position and orient the suction cup 406. The orientation system 424 is activatable through an actuation cable 425 controlled by a remote actuation controller 423. The remote actuation controller 423 may apply a force on a proximal part of the actuation cable 425 leading to a distal force which is transmitted to a wheel 426. The wheel is turned, inducing an orientation of the suction cup 406. The orientation system 424 may also comprise two wheels connected through a belt to ensure efficient deflection of the suction cup 406. The actuation cable 425 may be made of nitinol.

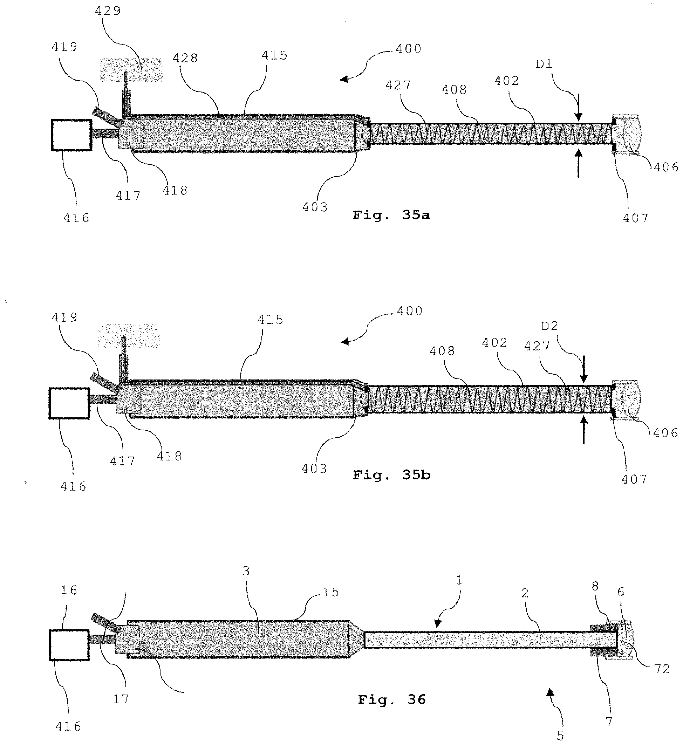

Fig. 35a shows a catheter device 400 having a distal section 402 which is expandable, in the unexpanded state having a diameter D1.

As shown in Fig. 35b, the distal section 402 is expanded so as to increase the diameter during an suction. The increased diameter D2 may help to prevent occlusion by thrombus fragments and to increase an aspiration flow rate to the proximal section. The first diameter D1 may be 300 um and the second diameter D2 may be 500 um. The expansive distal section comprises an expansive structure 427, made of nitinol wire with a diameter of 100 um coated with a polyurethane layer having a thickness of 200 um.

The expansive structure 427 is activated under a stimulus delivered through an actuation cable 428 controlled by a remote expansion controller 429. As an example, the remote expansion controller 429 may trigger a current which induces, by a Joule effect, the expansion of the expansive structure 427.

The nitinol structure used in some embodiments of the suction cup 406 may be used the expansive structure 427. The suction cup 406 may be expanded at the same time as the distal section 402 and/or may use the same expansive structure.

The catheter devices 400 shown herein may be moved manually or through proximal actuation. One or several proximal actuators, as shown herein may be combined with the catheter device 400, may be used to actuate the guidewire, the orientation of the suction cup, and/or the expansion of the distal section.

Fig. 36 shows a device 1 similar to the embodiment shown in Fig. 6a. Identical features denominated by identical reference numerals are not described repeatedly. Here, to avoid the occlusion of the flexible line 2, a filter 72 is placed inside in the suction cup 6. The filter 72 may be made of a metal, such as titanium or nitinol, or polymers, such as polypropylene, and/or a ceramic, or a combination of different materials.

The thickness of the filter 72 is between 50 um and 500 µm, preferably between 80 µm and 150 µm. The filter 72 may comprise holes, meshes, and/or bars. The size a filter opening hole may be between 300 µm² and 38000 µm².

It will be understood that a filter, as shown here, may also be used on any suction cup 406 of devices 400 shown in Figs. 31-35.

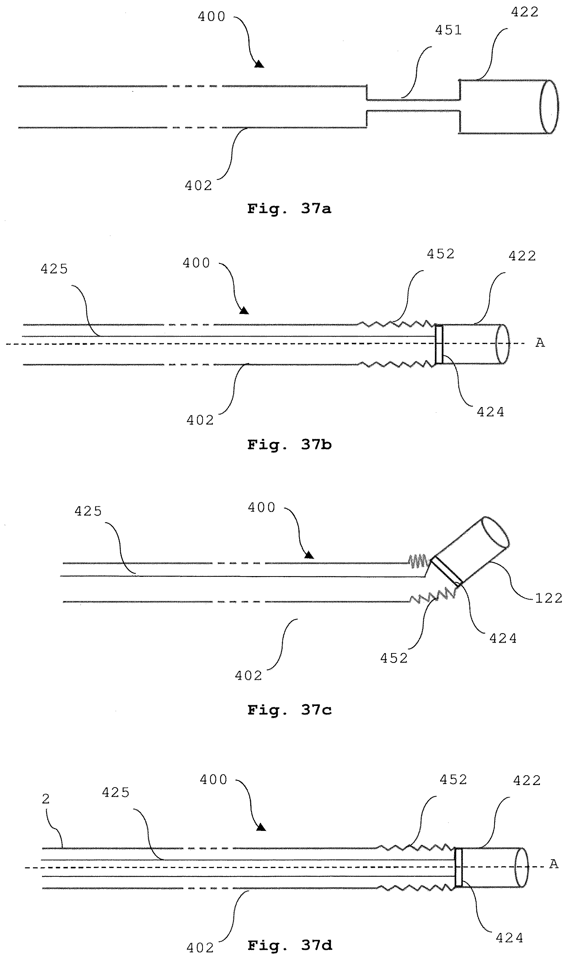

Figs. 37a-37f show the catheter device 1 further having an orientation segment 451; 452 which distinguishes the main body of the catheter device 1 from the distal part 122 which is to be reversibly displaced. The orientation segment can optionally be provided on the flexible line of the catheter device.

As seen in Fig. 37a, the orientation segment 451 has a reduced cross section compared to the cross section of the main body of the catheter device 400 as well as compared to the cross-section of the distal part 422 of the catheter device. The reduced cross section of the orientation segment may facilitate the transition of the distal part of the catheter from a first position to a deviated position. The orientation segment 451 having a reduced cross section may have a similar structure to the main body of the catheter. For instance, the orientation segment and the main body of the catheter may be layered structures. In this case, the orientation segment may have the same layers as the main body of the catheter but in a reduced size as compared to the main body of the catheter device. Alternatively, the orientation segment may have a different layer structure compared to the main body of the catheter device.

An exemplary structure of the orientation segment 451 may be made of an inner layer of PTFE, a nitinol coil and an outer layer of PEBA. The nitinol coil may be provided to allow more flexibility to the orientation segment. The thickness of the outer layer can be reduced to optimize the bending stiffness of the orientation segment, and may also be manufactured separately and assembled by gluing or welding.

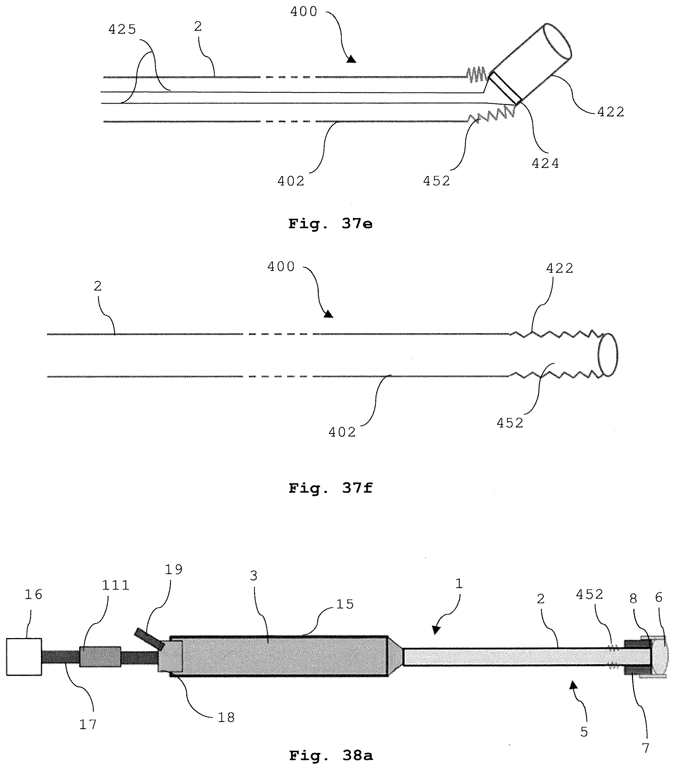

As depicted in fig. 37b-37f, the orientation segment 452 of some embodiments has a pleated profile. A pleated profile may enable a larger cross-section while retaining mechanical flexibility. Figs. 37b-37e shows the orientation segment 452 separating the main body of the catheter device 400 from the distal part 422 of the catheter device. As seen in Fig. 37f, the orientation segment 452 can be integrally formed with the distal part 422 of the catheter device 400. The pleated profile of the orientation segment 452 in figs. 37b-37f allows for flexible contracting and de-contracting of the orientation segment 452. The pleated orientation segment may optionally be made polyurethane and/or silicone.

Figure 37c and 37e shows respectively the orientation segment 452 of Fig. 37b and Fig. 37d after actuation of orientation system 424. The orientation system 424 is arranged at the distal part 422 of the catheter device, in particular, it is arranged near the orientation segment 452. It may be positioned anywhere along the distal part 422 of the catheter. It may optionally be coincident with the orientation segment 452. The orientation system 424 is actuated by a cable 425. The cable 425 can be actuated by pulling or pushing this introducing the translation of the cable. Here, the cable 425 retracts longitudinally causing a side of orientation segment 452 to contract. A bend is thus created such that the orientation segment 452, and therefore the attachment element (not shown), deviates from the main axis A of the catheter.

Referring to Figs. 37d and 37e, the orientation system 424 comprises two cables 425. Actuation of the orientation system 424 occurs via the translation of both cables in opposite directions. In other words, one cable retracts longitudinally, and the other cable extends longitudinally. The retracting cable causes the first side of orientation segment to which it is connected to contract (i.e. reduce the distance separating each pleat) whilst the extension of the second cable causes the opposing side to de-contract (i.e. increases the distance between each pleat). A bend is thus created such that the orientation segment 452, and therefore the attachment element (not shown), deviates from the main axis of the catheter (see also Fig. 32).

Fig. 38a shows a further embodiment of a device 1 having a main body 15 and a distal part 5 with a flexible line 2. The device 1 is substantially similar as the device shown in Fig. 6a, and identical features will not be described again for clarity. Here, the device further comprises a sensor 111. Here, the sensor 111 is a flow sensor measuring a pulling force on the distal part 5, allowing to determine the attachment of the suction cup 6 to a thrombus (not shown). Additionally or alternatively, a flow sensor, a temperature sensor, or a pressure sensor may also be used. The sensor 111 is arranged on a proximal end of the main body 15. The sensor 111 is arranged along connector 17 between the vacuum pump 16 and the access valve 18. The access valve 18 fluidly connects the vacuum pump 16/connector 17 assembly with the suction line 3 in the flexible line 2.

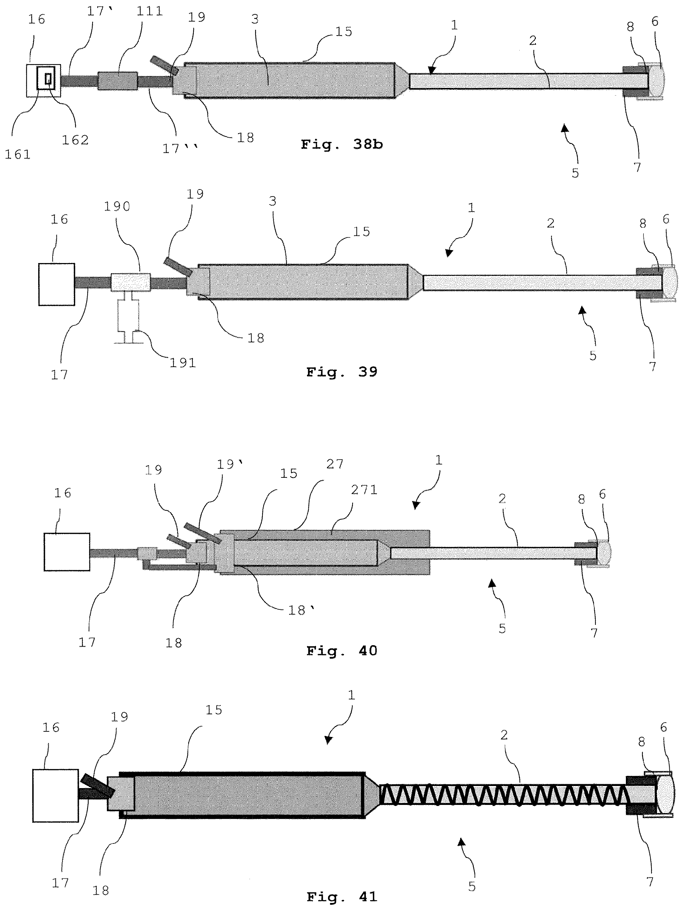

Fig. 38b shows a further embodiment of a device 1 having a main body 15 and a distal part 5 with a flexible line 2. The device 1 is substantially similar to the device shown in Fig. 38a, and identical features will not be described again for clarity. Here, the device vacuum pump comprises a trap 161 for capturing particles or substances from the catheter device. Here, two sensors 111; 162 are provided. The sensor 111 is a vacuum sensor measuring the level of suction within the catheter device. The second sensor is a trap sensor 162 positioned on the trap 161 of the vacuum pump 16. Sensor 111 is arranged along connector 17 and separates the connector into a first part 17' and second part 17''. The trap sensor 162 and the flow sensor 111 are in fluid connection with one another via the first part of the connector 17'. Sensor 111 is further connected to the access valve 18 via the second part of the connector 17''.

The first part of the connector 17' is provided with a smaller diameter than the diameter of the second part of the connector 17''. The difference in diameter between the first part 17' and second part 17" of the connector creates a pressure difference between the trap sensor 162 and the vacuum sensor 111 as the smaller diameter creates a higher resistance to the suction flow. This leads to a greater pressure drop for a given flow rate which results in even minor changes in flow rates are detected. Thus such a design allows for improved the sensitivity of the sensors such that even slight variations are detected which in turn means that even the slightest detaching of the thrombus from the suction cup may be detected.

Shown in Fig. 39 is a further embodiment of a device 1 having a main body 15 and a distal part 5 with a flexible line 2. The device 1 is substantially similar as the device shown in Fig. 6a, and identical features will not be described again for clarity. Here, the device further comprises a port 190 and a syringe 191. The port is arranged connector 17 between the vacuum pump 16 and the access valve 18. The access valve 18 fluidly connects the vacuum pump 16/connector 17 assembly with the suction line 3 in the flexible line 2. The syringe 191 attaches to the port 190 and is configured to monitor the attachment of the suction cup with the thrombus (not shown). To achieve this, the syringe 191 samples the flow. If blood is found in the flow, the thrombus is not sufficiently attached, and reattachment would be required. However, if blood is not detected in the flow, then the thrombus is sufficiently attached to the suction cup and may be extracted.

Fig. 40 depicts a further embodiment of a device 1 having a main body 15 and a distal part 5 with a flexible line 2. The device 1 is substantially similar as the device shown in Fig. 6a, and identical features will not be described again for clarity. Here, the device 1 further comprises a retrieving catheter 27; a second access valve 18'; a second port 19' and a second connector 173. The retrieving catheter 27 is configured to retain the thrombus (not shown) within its lumen 271 for extraction from the patient. The retrieving catheter 27 is adapted such that it surrounds a part of the catheter main body 15 and is equipped with an access valve 18' which has a connector 173 for the vacuum pump. -The access valve 18' fluidly connects the vacuum pump 16/connector 173 with the retrieving catheter lumen in which the catheter main body 15 is housed. The retrieving catheter access valve 18' further allows insertion of additional instruments such as a guidewire (not shown) or of a fluid via port 19' without entering thereto.

In a similar fashion to what is presented in Fig. 11, the medical device is retracted within the lumen of the retrieving catheter. To prevent detachment of the thrombus when the medical device is retracted within the retrieving catheter suction is provided within the lumen 271 of the retrieving catheter 27 to retrain the thrombus therewithin.

Alternatively, the retrieving catheter can provide aspiration during the retrieval of the thrombus with the medical device. This aspiration might reduce the blood flow in the vessel during the retrieval process.

Fig. 41 shows a further embodiment of a device 1 having a main body 15 and a distal part 5 with a flexible line 2. The device 1 is substantially similar as the device shown in Fig. 6a, and identical features will not be described again for clarity.

Here, the flexible line 2 comprises a reinforcement 2' extending along its length. The reinforcement is configured to prevent the flexible line 2 of the device from deforming and/or kinking during navigation. The reinforcement may increase the stiffness of the flexible line2 at its position. This allows the flexible line 2 to be flexible enough to be easily navigated through the vessel to the thrombus whilst also having the stiffness necessary to avoid twisting and kinking of the flexible line.

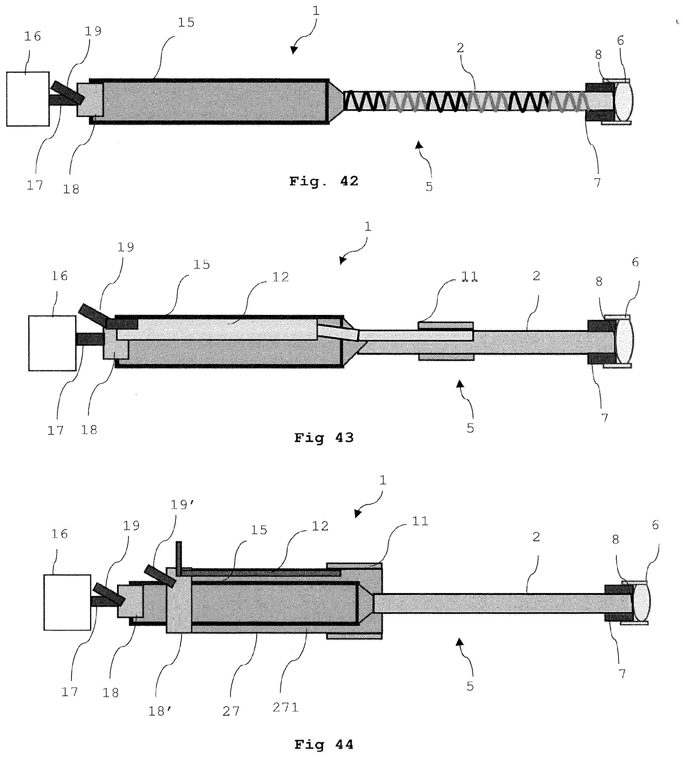

Fig. 42 illustrates a further embodiment of a device 1 having a main body 15 and a distal part 5 with a flexible line 2. The device 1 is substantially similar as the device shown in Fig. 6a, and identical features will not be described again for clarity. The device of Fig. 42 comprises a helical reinforcement 2' along the flexible line 2. The reinforcement comprises alternating radiopaque sections and non-radiopaque sections extending the length of the flexible line 2. It is conceivable, that sections may alternatively be a plurality of reinforcement elements. The radiopaque sections allow the visualisation of the flexible line as it is navigated within the patient. The radiopaque segment may be formed by a coil with a radiopaque ring or radiopaque coatings.

Fig. 43 shows a further embodiment of a device 1 having a main body 15 and a distal part 5 with a flexible line 2. The device 1 is substantially similar as the device, shown in Fig. 6a, and identical features will not be described again for clarity. The flexible line 2 comprises a balloon 11 which can be inflated by means of an inflation line 12. The inflation line passes through the access valve 18 and extends within the lumen of the catheter main body 15 and flexible line 2 to the balloon 11 to allow inflation of the balloon 11. The balloon 11 can be inflated with an inflation solution provided to the balloon via the inflation line1 2. It is conceivable that the inflation line 12 connects to an external container (not shown) which houses the inflation solution via the port 19.

Once inflated, the balloon 11 may reduce the blood flow in a vessel in an area where the balloon is located. The reduction of the blood flow may be advantageous both during positioning of the distal part 5 of the device 1 and during retrieval of the thrombus. The balloon may be only partially inflated to avoid a total stagnation of the flow.

Fig. 44 depicts a further embodiment of a device 1 having a main body 15 and a distal part 5 with a flexible line 2. The device 1 is substantially similar as the device shown in Fig. 6a, and identical features will not be described again for clarity. Here, the device 1 further comprises a retrieving catheter 27; a second access valve 18'; a second port 19'. The retrieving catheter 27 is configured to retain the thrombus (not shown) within its lumen 271 for extraction from the patient. The retrieving catheter 27 is adapted such that it surrounds a part of the catheter main body 15 and comprises an access valve 18'. The retrieving catheter 27 comprises a balloon 11 which can be inflated by means of an inflation line 12. When inflated, the balloon 11 may reduce the blood flow in a vessel in an area where the balloon is located. The reduction of the blood flow may be advantageous both during the positioning of the distal part 5 of the device 1 and during retrieval of the thrombus. The balloon 11, when inflated, may also provide stability to the catheter body 15 during treatment with the distal part 15. Furthermore, the balloon can be inflated using a contrast agent such that the location of the balloon may be clearly visualized by the practitioner thus improving the accuracy of the position of the distal part with regards to the thrombus.

The inflation line passes through the access valve 18' and extends within the lumen 271 of the retrieving catheter 27 to the balloon 11 to allow inflation of the balloon 11. The balloon 11 can be inflated with an inflation solution provided to the balloon via the inflation line 12. It is conceivable that the inflation line connects to an external container (not shown) which houses the inflation solution. The access valve 18' further allows insertion of additional instruments such as a guidewire (not shown) or of a fluid via port 19' without entering thereto. It is conceivable that the access port 18' may be further connected to a pump 16 in a similar fashion to what is presented in Fig. 40, such that suction may be applied within the lumen 271 of the retrieving catheter 27. In such a case, the medical device 1 may be retracted within the lumen 271 of the retrieving catheter 27 and suction may be provided within said lumen 271 of to retrain the thrombus therewithin.

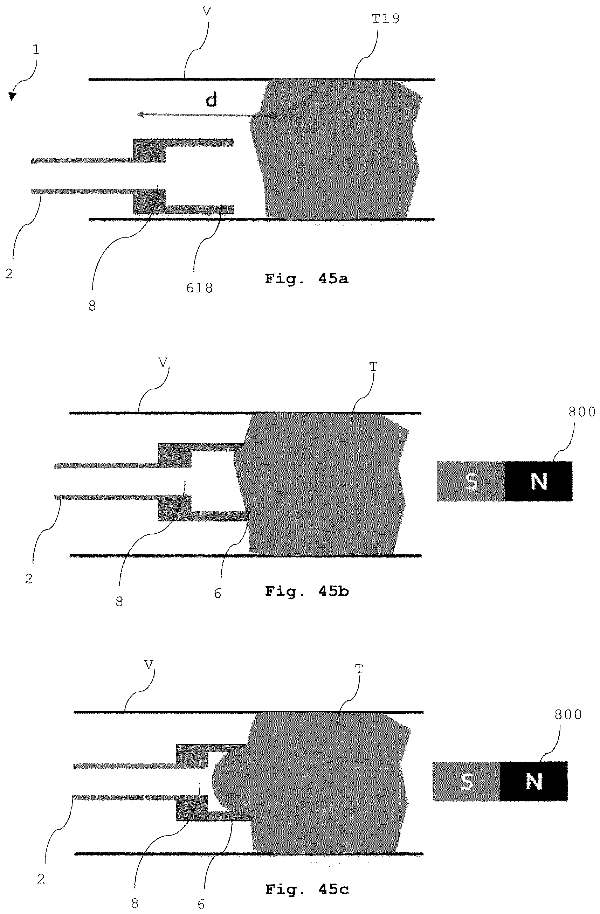

Fig. 45a-45c illustrates various steps of the method for monitoring the anchoring and retrieval of the thrombus. In Fig. 45a, the position of the thrombus T within the vessel V is determined. Knowing the position of the thrombus T allows for a target position of the suction cup to be determined by controlling unit (not shown). Preferably, the suction cup is positioned such that the distal part of the suction cup 6 is aligned with the proximal part of the catheter device, thereby ensuring a more accurate determintation of the position. The suction cup 6 is positioned at a distance d from the proximal surface of the thrombus. Once positioned, the distance between the suction cup 6 and the surface of the thrombus to which the suction cup is supposed to attach may be more accurately measured.

Fig. 45b shows the suction cup 6 being guided toward the surface if the thrombus, for example by the magnetic actuator 800. The suction cup is cup is parallel to the vessel. To allow navigation of the suction cup 6 the device is actuated such that the flexible line 2 is released leading to a slack in the flexible line 2. The measured distance between the suction cup 6 and the surface of the thrombus may be used to determine the amount of flexible line 2 that needs to be released to allow the suction cup 6 to be navigated such that it is in proximity to the surface of the thrombus for attachment thereto. It is conceivable that the amount of flexible line released may be greater than the measured distance between the suction cup 6 and the surface of the thrombus. For instance, the amount of flexible line released may be 1.5 times greater than the distance measured between the thrombus and the suction cup 6.

In some cases the anchoring of the suction cup 6 to the thrombus may need to be reperformed to ensure sufficient attachment on the suction cup 6 to the thrombus for retrieval. The suction cup 6 may be repositioned by operation of any one or more of: the magnetic actuator; the flexible line or a combination of flexible line and magnetic actuator. This step may be repeated as often as necessary to ensure the correct anchoring of the suction cup 6 to the thrombus.

The operator may reperform the positioning of the suction cup 6 with a new set of parameters with the current position of the suction cup 6 or reperform the attachment based of the initial target position of the suction cup 6.

Possible parameters may be: position of the suction cup 6 relative to the vessel (e.g. lumen, center line or height), tilt of the suction cup 6 (positive or negative according to the inner surface plan of the vessel), penetration of the suction cup 6 into the thrombus T.

The position and tilt of the suction cup 6 may be determined by using the magnetic actuator (position, displacement (continuous displacement for example). The penetration may be determined by the release of the medical device, the release of the medical device and the magnetic actuator, or the release of the medical device and the displacement of the magnetic actuator. The displacement of the magnetic actuator allows to determine the displacement of the magnetic field which might help the progression of the medical device. The system may switch to another sets of parameters which may be predetermined. Moreover, the user may propose an own set of parameters.

The operator can try anchoring without the magnetic actuator 800 during these steps. During the anchoring step, the delivery pump (not shown) of the medical device may be used to help the displacement of the suction cup 6, by removing proximal slack of the controlling line (e.g. straightening the controlling line), thereby moving/pushing the medical device forward. The control unit may compute the theoretical position of the catheter device, preferably the suction cup 6. Additionally, the operator and/or the control unit may be able to indicate the real position of the catheter device. The difference between the real and the theoretical position may be used to calculate the slack created with the flexible line during the anchoring.

Progression of the catheter device, preferably the suction cup 6, toward the proximal surface might be different according to the composition of the thrombus. In a thrombus having a high fibrin and platelet content (e.g. > 70%) the anchoring of the suction cup 6 will be reduced as compared to the anchoring of the suction cup 6 to a thrombus with a lower fibrin and platelet content. The difference between the theoretical position and the real position might provide information on the properties of the clot.

Fig. 45c show the anchoring of the suction cup 6 to the thrombus T. to achieve this suction is provided to the thrombus via a pump (not shown) and the opening 8 suction cup 6. The suction cup 6 attaches to the thrombus T via the suction. The medical device is then actuated such that the thrombus T is retrieved by a pulling motion toward the medical device. The theoretical position of the suction cup 6, during the retrieval step may be computed by the control unit (not shown). This theoretical position may be computed based on the slack provided to the flexible line 2 during the anchoring. Visualization of the theoretical position of the suction cup 6, may allow the operator to monitor the retrieving step, to evaluate any stretching of the flexible line. If significant stretching under stress is observed in the absence of displacement of the thrombus, it may be indicative of an unsuccessful retrieval.

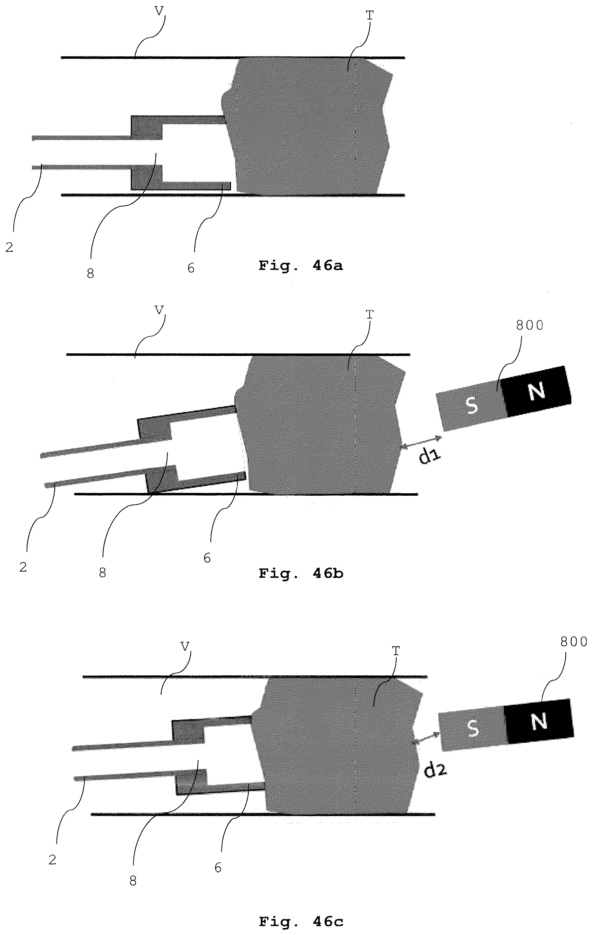

Fig. 46a- 46C illustrates the monitoring of the anchoring and retrieval of the thrombus. The method steps shown in fig. 46a and 46c are the same as those presented in Fig. 45a and 45c respectively.

Fig. 46b shows the orienting of the suction cup 6 to provide improved anchoring to the thrombus. The orienting of the suction cup is achieved by actuation of the magnetic actuator 800.

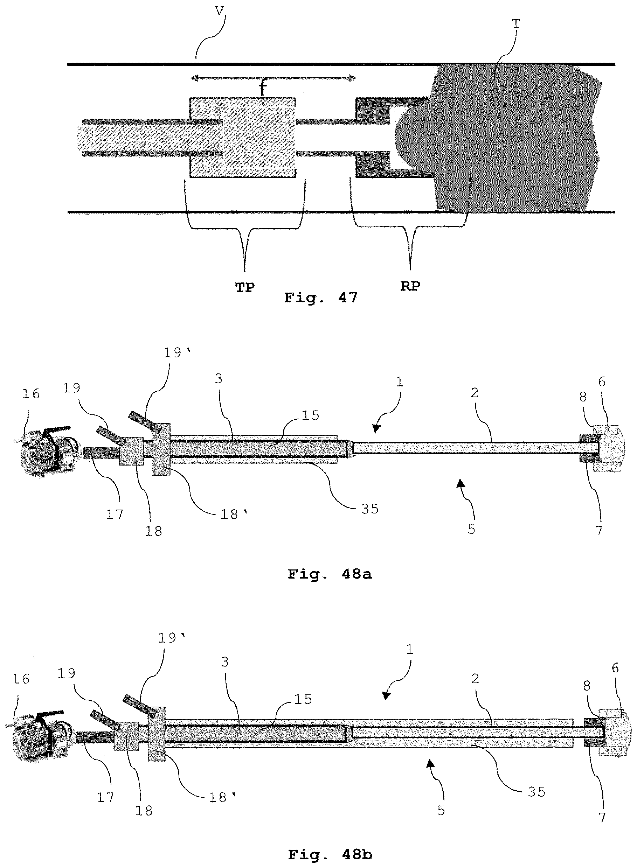

Fig. 47 shows the retrieval step of the method for monitoring the retrieval of the thrombus. Once the suction cup 6 has been anchored to the thrombus T, the theoretical position TP of the suction cup 6 is computed by the control unit (not shown). The actual position RP of the suction cup 6 is determined by the operator and provided to the control unit. The control unit computes the difference between the theoretical position TP and the actual position RP of the suction cup 6. The computed difference between the theoretical TP and actual position RP is then used to indicate the slack or the stretching of the flexible line 2 according to the progression of the retrieving. Suction is applied to the thrombus T via the opening 8 and suction cup 6

Fig. 48a shows a further embodiment of a catheter device 1 according to the invention. The catheter device 1 shown here is similar to the embodiment of Fig. 6a, and identical features will not be described again for clarity. Here, the catheter device additionally has a second access valve 18' and a second port 19', similar to, e.g., the embodiment of Fig. 44. The catheter device 1 further has an extendable distal support 35. As shown in Fig. 48a, the distal support may be retracted and substantially coincide with the catheter body 15. When extended (see Fig. 48b), the distal support 35 covers a majority of the flexible line 2 of the catheter. device 1.

During the retrieving, the flexible line 2 may be stretched. The stretching may cause displacement or stress of the vessel/vessel wall. The distal support 35 may reduce such displacements/stress and may, for example, be formed by a neurovascular catheter with a fixed length which may be moved over the flexible line 2. An inner diameter of the distal support 35 may be above the outer diameter of the catheter device 1, in particular the main body 15 and/or the flexible line 2. An outer diameter of the distal support 35 may be smaller than an outer diameter of the magnetic element 7 or the suction cup 6, thereby limiting the thickness of the distal support 35 and reducing the bending stiffness and improving the progression of the distal support 35 in a tortuous anatomy. The distal support 35 may have a breaking strain below 200%, preferably below 100%. The distal support 35 may be made of inner lining PTFE, a coil, an outer jacket and optionally an outer coating. The inner diameter is 1.1 mm and the outer diameter is 1.4 mm. A magnetic cup having an outer diameter of 1.5 mm and device diameter of 0.9 mm may be used in this embodiment.

Figs. 49a-49f show method of attaching to a thrombus T and retrieving said thrombus T. The method is similar to the method shown in Figs. 27a-27h. Here, no guidewire is present. Additionally, the medical device 1 is smaller than the medical device of Figs. 27a-27h.

Generally, the medical device may comprises at least a line, preferably a flexible for the flow-driven navigation, and an attachment element. Preferably, the medical device comprises a proximal rigid element, a flexible line, a magnetic element and a suction cup. The medical device may be navigated up to the thrombus and then anchored to the thrombus by inducing the vacuum. The medical device is preferably anchored in the center of the thrombus under magnetic guidance by the magnetic actuator. Once the medical device is anchored, an aspiration catheter may be moved over the medical device up to the thrombus. Preferably, a slight vacuum is done in the aspiration catheter to anchor it to the thrombus at the appropriate position. The medical device may then be moved backward over at least 1 mm, preferably between 1 mm and 10 mm. Then the vacuum may be stopped in the medical device. The medical device may be removed from the aspiration catheter. Preferably, the vacuum in the aspiration is increased up to - 0.99 bar, preferably up to -0.9 bar. Preferably, after a waiting time of 2 min, the aspiration catheter is moved backward with the thrombus.

## Claims

1. A catheter device (1) for retrieving a thrombus (T) from a vessel, comprising
a main body (15) with an internal channel (15') extending at least through a distal portion of the main body (15),
an attachment element (4), preferably attached or attachable to the main body (15) by a flexible line (2),
wherein the attachment element (4) is adapted to attach to the thrombus (T) and further wherein the catheter device comprises an orientation segment (451; 452) configured for revertible orientation of the attachment element (4) with respect to the main body (15).

2. The catheter device (1) according to claim 1, wherein the orientation segment (451; 452) is positioned upstream to the distal portion of the main body or is coincidental with the distal portion.

3. The catheter device (1) according to any one of the preceding claims, wherein the orientation segment (451; 452) has a reduced cross section with respect to the main body (15) and/or a reduced cross section with respect to the distal portion of the main body (15).

4. The catheter device (1) according to any one of the preceding claims, wherein the orientation segment (452) has a pleated profile.

5. The catheter device (1) according to any proceeding claims, wherein the orientation segment (451; 452) comprises a flexible material and/or a shape memory material.

6. The catheter device (1) according to claim 5, wherein the flexible material comprises any one or more of a polymer, a metal, a resin, a rubber.

7. The catheter device (1) according to claim 5, wherein the shape memory material comprises any one or more of a shape memory polymer and/or a shape memory alloy.

8. The catheter device (1) according to any proceeding claims, further comprising an orientation system (424) configured to bend the orientation segment (451; 452).

9. The catheter device (1) according to claim 8, wherein the orientation system (424) comprises an activation component (425) for bending the orientation segment (451; 452).

10. The catheter device according to claim 9, wherein the activation component (425) comprises any one or more of a cable, a wire, a coil, a spring, a magnet.

11. The catheter device (1) according to any one of the preceding claims, wherein the orientation system (424) comprises a magnetic element (7) for steering and/or guiding by an external magnetic actuator.

12. The catheter device (1) according to any one of the preceding claims, wherein the attachment element (4) comprises a magnetic element (7) for steering and/or guiding by an external magnetic actuator and suction cup (6).

13. The catheter device (1) according to any one of the preceding claims, preferably wherein the suction cup (6) is rigidly attached to the magnetic element (7) and the magnetic element (7) is adapted to be orientable by an external magnetic field.

14. The catheter device (1) according to any proceeding claims, wherein the orientation segment (6) is orientable by a means of a magnetic actuator.

15. The catheter device (1) according to any one of the preceding claims, wherein the main body (15) comprises a rigid portion, preferably a rigid portion forming a distal-most portion of the main body (15), said rigid portion being downstream of the orientation segment (452).

16. The catheter device (1) according to any one of the preceding claims, further comprising a sensor (9; 111; 162) configured to monitor the attachment of the attachment element to the thrombus.

17. The catheter device (1) according to any one of the preceding claims, further comprising a plurality of sensors (9; 111; 162) configured to monitor the attachment of the attachment element to the thrombus.

18. The catheter device (1) according to claim 16 or 17,
wherein the sensor (9; 111; 162) is any one or more of a force sensor; an electrical sensor; a flow sensor; a vacuum sensor.

19. The catheter device (1) according to any one of the preceding claims, further comprising a syringe (171) configured to monitor the attachment of the attachment element to the thrombus.

20. The catheter device (1) according to any one of the preceding claims, further comprising a retrieving catheter (27) configured for extraction the thrombus.

21. A system (200) comprising a first catheter device, the first catheter device being a catheter device (1) according to any one of the preceding claims, and a second catheter device which is a retrieving catheter (212), wherein the second catheter device is adapted to be delivered along the first catheter device, further wherein the first catheter device is adapted to be retrieved through an inner channel of the second catheter device, and wherein the second catheter device is adapted to provide an aspiration for removing a thrombus (T).

22. The system according to claim 20, comprising at least one vacuum pump (16) configured to provides aspiration in at least one of the first catheter device or the second catheter device.

23. The catheter device (1) according to any one of claims 1 to 20 and/or the system according to any one of claim 21 or 22, wherein the at least one catheter device (1) is configured for remote actuation; preferably remote magnetic actuation.
